# EUROPEAN PATENT APPLICATION

(11) **EP 4 374 875 A1**
(43) Date of publication of application: **29.05.2024**
(21) Application number: 22845446.8
(22) Date of filing: 22.07.2022
(51) Int. Cl.: A61K 39/395, A61K 31/4709, A61P 35/00

(54) **COMBINATION DRUG FOR TREATMENT OF GASTRIC CARCINOMA AND/OR ESOPHAGOGASTRIC JUNCTION CANCER**

(30) Priority: 22.07.2021 CN 202110831932
(71) Applicant: CHIA TAI TIANQING PHARMACEUTICAL GROUP CO., LTD., Lianyungang, Jiangsu 222062 (CN)
(72) Inventor: LUO, Suxia, Zhengzhou, Henan 450003 (CN); LI, Ning, Zhengzhou, Henan 450003 (CN); MA, Yijie, Zhengzhou, Henan 450003 (CN); BIE, Liangyu, Zhengzhou, Henan 450003 (CN); LI, Jiajun, Lianyungang, Jiangsu 222062 (CN)
(74) Representative: WSL Patentanwälte Partnerschaft mbB
(86) International application number: PCT/CN2022/107363
(87) International publication number: WO 2023/001283

(57) **Abstract**

A combination drug for treatment of gastric carcinoma and/or esophagogastric junction cancer, comprising an anti-PD-L1 antibody and anlotinib or a pharmaceutically acceptable salt thereof. The combination drug further comprises at least one third therapeutic agent. In addition, the present application further provides a use of a combination drug or a pharmaceutic composition, which comprises an anti-PD-L1 antibody and anlotinib or a pharmaceutically acceptable salt thereof, in preparation of a drug for treatment of gastric carcinoma and/or esophagogastric junction cancer.

## Description

### TECHNICAL FIELD

The present application relates to the field of biomedicine, and particularly to a pharmaceutical combination for treating gastric carcinoma and/or esophagogastric junction cancer.

### BACKGROUND

Gastric carcinoma is a malignant tumor originating from the epithelium of the gastric mucosa, which is the most common malignant tumor of the digestive tract and is one of the leading causes of cancer deaths worldwide. It is estimated that more than half of the cases and deaths of gastric carcinoma occur in China. In 2015, there were about 679,000 new cases and 498,000 deaths of gastric carcinoma in China, making gastric carcinoma the second most common cancer after lung cancer.

At present, surgery plays a dominant role among the treatments for gastric carcinoma, and it is also an important treatment method that can achieve the purpose of cure at present. However, because the early diagnosis rate of gastric carcinoma in China is low, the 5-year survival rate after surgery is only 20%-30%. Therefore, most patients with gastric carcinoma are difficult to cure by surgery alone, and they also need to receive multimodality therapy such as chemotherapy and radiotherapy. In addition, patients with locally advanced gastric carcinoma may create conditions for surgical resection after preoperative chemotherapy, and patients at high risk of recurrence and metastasis after surgery need to receive postoperative adjuvant chemotherapy. Therefore, chemotherapy still plays an important role in the multimodality therapy of gastric carcinoma. At present, the first-line treatment for advanced gastric carcinoma is mainly a two- or three-drug chemotherapy regimen based on a fluorouracil anti-tumor drug (5-FU, capecitabine, or tegafur-gimeracil-oteracil potassium) in combination with platinum (oxaliplatin or cisplatin) and/or paclitaxel anti-tumor drugs, but the median overall survival (mOS) does not exceed 1 year. Meanwhile, most patients with gastric carcinoma cannot tolerate subsequent treatment due to deterioration of their conditions. First-line treatment is the best chance for the patients with gastric carcinoma to achieve a curative effect, so that new therapeutic agents or treatment methods need to be actively explored and researched.

Furthermore, esophagogastric junction (EGJ) refers to an area specifically designated by a virtual anatomical junction between the esophagus and the stomach, which can be located in the following ways: the narrowest area of the lower esophagus in a barium meal examination of the digestive tract; under endoscopy, the longitudinal palisade-like blood vessel of the lower esophagus or the upper edge line of the longitudinal folds of the gastric proximal end; pathologically, the junction area between squamous epithelium and columnar epithelium; on a gross specimen, the area where the diameter of the esophagus and gastric lumen changes. In recent years, the incidence rate of esophagogastric junction cancer, especially adenocarcinoma of esophagogastric junction (AEG), has been increasing. Data from a long-term follow-up study in China show that, for AEG patients, the 5-year survival rate is about 30% and the 10-year survival rate is only 13.3%. Therefore, it is of great significance to actively explore and research new therapeutic agents or treatment methods for esophagogastric junction cancer.

### SUMMARY

In one aspect, the present application provides a pharmaceutical combination for treating gastric carcinoma and/or esophagogastric junction cancer, which comprises: an anti-PD-L1 antibody, and anlotinib or a pharmaceutically acceptable salt thereof. In some embodiments, the pharmaceutical combination further comprises at least one third therapeutic agent, wherein the third therapeutic agent is one or more of a fluorouracil anti-tumor drug, a platinum anti-tumor drug, and a paclitaxel anti-tumor drug. In some embodiments, the fluorouracil anti-tumor drug includes, but is not limited to, one or more selected from the group consisting of capecitabine, 5-fluorouracil, difuradin, doxifluridine, trifluridine, tegafur, carmofur, tegafur-gimeracil-oteracil potassium, and uracil-tegafur; the platinum anti-tumor drug includes, but is not limited to, one or more selected from the group consisting of miriplatin, cisplatin, carboplatin, dicycloplatin, nedaplatin, oxaliplatin, lobaplatin, triplatin tetranitrate, phenanthriplatin, picoplatin, and satraplatin; and the paclitaxel anti-tumor drug includes, but is not limited to, one or more selected from the group consisting of paclitaxel, paclitaxel liposome, albumin-bound paclitaxel, and docetaxel. In some embodiments, the third therapeutic agent comprises a fluorouracil anti-tumor drug and/or a platinum anti-tumor drug. In some embodiments, the third therapeutic agent comprises capecitabine and/or a platinum anti-tumor drug. In some embodiments, the third therapeutic agent comprises a fluorouracil anti-tumor drug and/or oxaliplatin. In some embodiments, the third therapeutic agent comprises capecitabine and/or oxaliplatin. In some embodiments, the third therapeutic agent comprises capecitabine and/or cisplatin. In some embodiments, the third therapeutic agent comprises 5-fluorouracil and/or oxaliplatin. In some embodiments, the anti-PD-L1 antibody is an anti-PD-L1 humanized monoclonal antibody. In some embodiments, the third therapeutic agent comprises: one or more selected from the group consisting of capecitabine, 5-fluorouracil, difuradin, doxifluridine, trifluridine, tegafur, carmofur, tegafur-gimeracil-oteracil potassium, and uracil-tegafur; and at least one selected from the group consisting of cisplatin, carboplatin, nedaplatin, and oxaliplatin. In some embodiments, the third therapeutic agent comprises: capecitabine, and at least one selected from the group consisting of cisplatin, carboplatin, nedaplatin, and oxaliplatin.

In some embodiments, the present application provides a pharmaceutical combination for treating non-HER2-positive gastric carcinoma and/or esophagogastric junction cancer, which comprises: an anti-PD-L1 antibody, and anlotinib or a pharmaceutically acceptable salt thereof. In some embodiments, the pharmaceutical combination further comprises at least one third therapeutic agent as described above.

In some embodiments, the pharmaceutical combination further comprises a pharmaceutically acceptable carrier.

In another aspect, the present application further provides a pharmaceutical combination for treating gastric carcinoma and/or esophagogastric junction cancer, which comprises a first pharmaceutical combination administered to a patient in need thereof during an initial treatment and, optionally, a second pharmaceutical combination administered to a patient in need thereof during a maintenance treatment, wherein the first pharmaceutical combination comprises an anti-PD-L1 antibody, and anlotinib or a pharmaceutically acceptable salt thereof, and at least one third therapeutic agent, the third therapeutic agent being one or more of a fluorouracil anti-tumor drug, a platinum anti-tumor drug, and a paclitaxel anti-tumor drug; and the second pharmaceutical combination comprises an anti-PD-L1 antibody, and anlotinib or a pharmaceutically acceptable salt thereof. In some embodiments, the third therapeutic agent comprises a fluorouracil anti-tumor drug and/or a platinum anti-tumor drug. In some embodiments, the third therapeutic agent comprises capecitabine and/or a platinum anti-tumor drug. In some embodiments, the third therapeutic agent comprises a fluorouracil anti-tumor drug and/or oxaliplatin. In some embodiments, the third therapeutic agent comprises capecitabine and/or oxaliplatin. In some embodiments, the third therapeutic agent comprises capecitabine and/or cisplatin. In some embodiments, the third therapeutic agent comprises 5-fluorouracil and/or oxaliplatin. In some embodiments, the initial treatment comprises 0 to 10 treatment cycles, preferably 1 to 10 treatment cycles, more preferably 1 to 6 treatment cycles, and most preferably 6 treatment cycles. In some embodiments, the initial treatment comprises 0 to 10 consecutive treatment cycles, preferably 1 to 10 consecutive treatment cycles, more preferably 1 to 6 consecutive treatment cycles, and most preferably 6 consecutive treatment cycles, from the first dose. Herein, the maintenance treatment is a treatment performed after the initial treatment.

In some embodiments, the present application provides a pharmaceutical combination for treating non-HER2-positive gastric carcinoma and/or esophagogastric junction cancer, which comprises a first pharmaceutical combination administered to a patient in need thereof during an initial treatment and, optionally, a second pharmaceutical combination administered to a patient in need thereof during a maintenance treatment, wherein the first pharmaceutical combination comprises an anti-PD-L1 antibody, and anlotinib or a pharmaceutically acceptable salt thereof, and at least one third therapeutic agent, the third therapeutic agent being one or more of a fluorouracil anti-tumor drug, a platinum anti-tumor drug, and a paclitaxel anti-tumor drug; and the second pharmaceutical combination comprises an anti-PD-L1 antibody, and anlotinib or a pharmaceutically acceptable salt thereof. In some embodiments, the initial treatment comprises 0 to 10 treatment cycles, preferably 1 to 10 treatment cycles, more preferably 1 to 6 treatment cycles, and most preferably 6 treatment cycles. In some embodiments, the initial treatment comprises 0 to 10 consecutive treatment cycles, preferably 1 to 10 consecutive treatment cycles, more preferably 1 to 6 consecutive treatment cycles, and most preferably 6 consecutive treatment cycles, from the first dose.

In some embodiments, the pharmaceutical combination further comprises a pharmaceutically acceptable carrier. In still another aspect, the present application provides use of a pharmaceutical combination comprising an anti-PD-L1 antibody, and anlotinib or a pharmaceutically acceptable salt thereof for preparing a medicament for treating gastric carcinoma and/or esophagogastric junction cancer. In some embodiments, the present application provides use of a pharmaceutical combination comprising an anti-PD-L1 antibody, and anlotinib or a pharmaceutically acceptable salt thereof for preparing a medicament for treating non-HER2-positive gastric carcinoma and/or esophagogastric junction cancer. In some embodiments, the pharmaceutical combination further comprises at least one third therapeutic agent, wherein the third therapeutic agent is one or more of a fluorouracil anti-tumor drug, a platinum anti-tumor drug, and a paclitaxel anti-tumor drug.

In yet another aspect, the present application provides use of a pharmaceutical combination comprising an anti-PD-L1 antibody, and anlotinib or a pharmaceutically acceptable salt thereof for treating gastric carcinoma and/or esophagogastric junction cancer. In some embodiments, the present application provides use of a pharmaceutical combination comprising an anti-PD-L1 antibody, and anlotinib or a pharmaceutically acceptable salt thereof for treating non-HER2-positive gastric carcinoma and/or esophagogastric junction cancer. In some embodiments, the pharmaceutical combination further comprises at least one third therapeutic agent, wherein the third therapeutic agent is one or more of a fluorouracil anti-tumor drug, a platinum anti-tumor drug, and a paclitaxel anti-tumor drug.

In yet another aspect, the present application further provides a method for treating gastric carcinoma and/or esophagogastric junction cancer, which comprises administering to a patient in need thereof a therapeutically effective amount of an anti-PD-L1 antibody, and anlotinib or a pharmaceutically acceptable salt thereof. In some embodiments, the method further comprises administering to a patient in need thereof a therapeutically effective amount of at least one third therapeutic agent. In some embodiments, the method comprises: administering the anti-PD-L1 antibody, and anlotinib or the pharmaceutically acceptable salt thereof, and the third therapeutic agent for an initial treatment; and then optionally administering the anti-PD-L1 antibody, and anlotinib or the pharmaceutically acceptable salt thereof for a maintenance treatment. In some embodiments, the third therapeutic agent is one or more of a fluorouracil anti-tumor drug, a platinum anti-tumor drug, and a paclitaxel anti-tumor drug. In some embodiments, the third therapeutic agent comprises a fluorouracil anti-tumor drug and/or a platinum anti-tumor drug. In some embodiments, the third therapeutic agent comprises capecitabine and/or a platinum anti-tumor drug. In some embodiments, the third therapeutic agent comprises a fluorouracil anti-tumor drug and/or oxaliplatin. In some embodiments, the third therapeutic agent comprises capecitabine and/or oxaliplatin.

In some embodiments, the application further provides a method for treating non-HER2-positive gastric carcinoma and/or esophagogastric junction cancer, which comprises administering to a patient in need thereof a therapeutically effective amount of an anti-PD-L1 antibody, and anlotinib or a pharmaceutically acceptable salt thereof. In some embodiments, the method further comprises administering to a patient in need thereof a therapeutically effective amount of at least one third therapeutic agent. In some embodiments, the method comprises: administering the anti-PD-L1 antibody, and anlotinib or the pharmaceutically acceptable salt thereof, and the third therapeutic agent for an initial treatment; and then optionally administering the anti-PD-L1 antibody, and anlotinib or the pharmaceutically acceptable salt thereof for a maintenance treatment.

In some embodiments, the anti-PD-L1 antibody, and anlotinib or the pharmaceutically acceptable salt thereof can be administered simultaneously, nonsimultaneously, or sequentially. In some embodiments, the anti-PD-L1 antibody, anlotinib or the pharmaceutically acceptable salt thereof, and the third therapeutic agent can be administered simultaneously, nonsimultaneously, or sequentially. In some embodiments, the present application further provides a method for treating gastric carcinoma and/or esophagogastric junction cancer, which comprises administering to a patient in need thereof a therapeutically effective amount of a pharmaceutical combination comprising an anti-PD-L1 antibody, and anlotinib or a pharmaceutically acceptable salt thereof. In some embodiments, the present application further provides a method for treating non-HER2-positive gastric carcinoma and/or esophagogastric junction cancer, which comprises administering to a patient in need thereof a therapeutically effective amount of a pharmaceutical combination comprising an anti-PD-L1 antibody, and anlotinib or a pharmaceutically acceptable salt thereof. In some embodiments, the pharmaceutical combination further comprises at least one third therapeutic agent. In some embodiments, the third therapeutic agent is one or more of a fluorouracil anti-tumor drug, a platinum anti-tumor drug, and a paclitaxel anti-tumor drug.

In yet another aspect, the present application provides a kit for treating gastric carcinoma and/or esophagogastric junction cancer or a kit for treating non-HER2-positive gastric carcinoma and/or esophagogastric junction cancer, which comprises: an anti-PD-L1 antibody, and anlotinib or a pharmaceutically acceptable salt thereof. In some embodiments, the anti-PD-L1 antibody is contained in a first compartment, and anlotinib or the pharmaceutically acceptable salt thereof is contained in a second compartment; they can be administered to a patient in need thereof simultaneously, nonsimultaneously, or sequentially. In some embodiments, the kit further comprises instructions for combined use of the anti-PD-L1 antibody, and anlotinib or the pharmaceutically acceptable salt thereof. In some embodiments, the kit comprises: a pharmaceutical composition of the anti-PD-L1 antibody, and a pharmaceutical composition of anlotinib or the pharmaceutically acceptable salt thereof.

In some embodiments, the present application provides a kit for treating gastric carcinoma and/or esophagogastric junction cancer or a kit for treating non-HER2-positive gastric carcinoma and/or esophagogastric junction cancer, which comprises: an anti-PD-L1 antibody, and anlotinib or a pharmaceutically acceptable salt thereof, and at least one third therapeutic agent. In some embodiments, the anti-PD-L1 antibody is contained in a first compartment, anlotinib or the pharmaceutically acceptable salt thereof is contained in a second compartment, and the third therapeutic agent is contained in an additional compartment; optionally, the number of the compartments in the kit can be increased as appropriate depending on the type of the third therapeutic agent; the anti-PD-L1 antibody, anlotinib or the pharmaceutically acceptable salt thereof, and the at least one third therapeutic agent can be administered to a patient in need thereof simultaneously, nonsimultaneously, or sequentially. In some embodiments, the kit further comprises instructions for combined use of the anti-PD-L1 antibody, anlotinib or the pharmaceutically acceptable salt thereof, and the third therapeutic agent. In some embodiments, the kit comprises: a pharmaceutical composition of the anti-PD-L1 antibody, a pharmaceutical composition of anlotinib or the pharmaceutically acceptable salt thereof, and a pharmaceutical composition of the third therapeutic agent.

In some embodiments, the pharmaceutical combination comprising the anti-PD-L1 antibody and anlotinib or the pharmaceutically acceptable salt thereof described herein comprises: a pharmaceutical composition of the anti-PD-L1 antibody, and a pharmaceutical composition of anlotinib or the pharmaceutically acceptable salt thereof. In some embodiments, the pharmaceutical combination further comprises a pharmaceutical composition of the at least one third therapeutic agent. In some embodiments, the third therapeutic agent comprises capecitabine and/or oxaliplatin. In one specific embodiment, the pharmaceutical combination comprising the anti-PD-L1 antibody and anlotinib or the pharmaceutically acceptable salt thereof described herein comprises: a pharmaceutical composition of the anti-PD-L1 antibody, a pharmaceutical composition of anlotinib or the pharmaceutically acceptable salt thereof, a pharmaceutical composition of capecitabine, and a pharmaceutical composition of oxaliplatin.

In some embodiments, the pharmaceutical composition further comprises a pharmaceutically acceptable carrier.

In some embodiments, the pharmaceutical combination comprising the anti-PD-L1 antibody and anlotinib or the pharmaceutically acceptable salt thereof described herein comprises: a pharmaceutical composition comprising 600-2400 mg of the anti-PD-L1 antibody. In some embodiments, the pharmaceutical composition of the anti-PD-L1 antibody is in a unit dose or in multiple doses.

In some embodiments, the pharmaceutical combination comprising the anti-PD-L1 antibody and anlotinib or the pharmaceutically acceptable salt thereof described herein comprises: a pharmaceutical composition comprising 6-12 mg of anlotinib or the pharmaceutically acceptable salt thereof. In some embodiments, the pharmaceutical composition of anlotinib or the pharmaceutically acceptable salt thereof is administered in a unit dose of 6 mg, 8 mg, 10 mg, and/or 12 mg.

In some embodiments, the pharmaceutical combination comprising the anti-PD-L1 antibody and anlotinib or the pharmaceutically acceptable salt thereof described herein comprises: a pharmaceutical composition comprising 600-2400 mg of the anti-PD-L1 antibody, and a pharmaceutical composition comprising 6 mg, 8 mg, 10 mg, and/or 12 mg of anlotinib or the pharmaceutically acceptable salt thereof in a unit dose, wherein the pharmaceutical composition of the anti-PD-L1 antibody is in a unit dose or in multiple doses. In some embodiments, the pharmaceutical combination comprises: a pharmaceutical composition comprising 600-2400 mg of the anti-PD-L1 antibody provided in a multiple-dose form, and a pharmaceutical composition comprising 6 mg, 8 mg, 10 mg, and/or 12 mg of anlotinib or the pharmaceutically acceptable salt thereof in a unit dose. In some embodiments, the pharmaceutical combination is a formulation suitable for administration within a single treatment cycle (e.g., a treatment cycle of 21 days), and comprises: a pharmaceutical composition comprising 600-2400 mg of the anti-PD-L1 antibody and a pharmaceutical composition comprising 84-168 mg of anlotinib or the pharmaceutically acceptable salt thereof.

In some embodiments, the formulation suitable for administration within a single treatment cycle (e.g., a treatment cycle of 21 days) further comprises: a pharmaceutical composition comprising 65-780 mg of oxaliplatin, and/or a pharmaceutical composition comprising 14000-168000 mg of capecitabine.

In some embodiments, the pharmaceutical combination comprising the anti-PD-L1 antibody and anlotinib or the pharmaceutically acceptable salt thereof described herein comprises: an anti-PD-L1 antibody and anlotinib or a pharmaceutically acceptable salt thereof in a weight ratio of (0.35-29):1, (3.5-29):1, (3.5-14.5):1, or (7-14.5):1, wherein the anti-PD-L1 antibody, and anlotinib or the pharmaceutically acceptable salt thereof can be packaged separately or packaged together; anlotinib can be packaged in multiple aliquots (e.g., 2 aliquots, 7 aliquots, 14 aliquots, 28 aliquots, or more); and the anti-PD-L1 antibody can be packaged in a single aliquot or in multiple aliquots (e.g., 2 aliquots, 4 aliquots, or more). In some embodiments, the pharmaceutical combination further comprises oxaliplatin and/or capecitabine, wherein the anti-PD-L1 antibody, anlotinib or the pharmaceutically acceptable salt thereof, oxaliplatin, and capecitabine are in a weight ratio of (600-2400):(84-168):(65-780):(14000-168000). Oxaliplatin and capecitabine can be packaged in a single aliquot or in multiple aliquots (e.g., 2 aliquots, 4 aliquots, or more).

In some embodiments, in the pharmaceutical combination comprising the anti-PD-L1 antibody and anlotinib or the pharmaceutically acceptable salt thereof described herein, the anti-PD-L1 antibody and anlotinib or the pharmaceutically acceptable salt thereof are each in the form of a pharmaceutical composition and can be administered simultaneously, nonsimultaneously, or sequentially; and
the amount of the anti-PD-L1 antibody administered can be determined according to the severity of the disease, the response of the disease, any treatment-related toxicity, and the age and health of a patient; for example, the anti-PD-L1 antibody may be administered at a daily dose of 600-2400 mg. In some embodiments, the anti-PD-L1 antibody may be administered at a daily dose of 600 mg, 800 mg, 1000 mg, 1200 mg, 1400 mg, 1600 mg, 1800 mg, 2000 mg, 2200 mg, or 2400 mg. In some embodiments, the anti-PD-L1 antibody is administered parenterally. In some embodiments, the anti-PD-L1 antibody is administered intravenously. In some embodiments, the pharmaceutical composition of the anti-PD-L1 antibody is at a concentration of 10-60 mg/mL. In some embodiments, the pharmaceutical composition of the anti-PD-L1 antibody is at a concentration of 10 mg/mL, 20 mg/mL, 30 mg/mL, 40 mg/mL, 50 mg/mL, or 60 mg/mL.

The administration regimen for the anti-PD-L1 antibody can be determined comprehensively depending on the activity and toxicity of the drug, the tolerance of a patient, and the like. In some embodiments, for the anti-PD-L1 antibody, every 1 week, every 2 weeks, every 3 weeks, or every 4 weeks is counted as one treatment cycle. In some embodiments, the anti-PD-L1 antibody is administered once a week, every 2 weeks, every 3 weeks, or every 4 weeks. In some embodiments, the anti-PD-L1 antibody is administered at a dose of 600-2400 mg per treatment cycle. In some specific embodiments, the anti-PD-L1 antibody is administered at a dose of 1200 mg per treatment cycle. In some embodiments, the anti-PD-L1 antibody is administered once every 3 weeks at a dose of 600-2400 mg each time. In some embodiments, anlotinib or the pharmaceutically acceptable salt thereof is administered at a daily dose of 6 mg, 8 mg, 10 mg, or 12 mg on a regimen of consecutively 2-week treatment and then 1-week interruption.

In some embodiments, the pharmaceutical combination comprising the anti-PD-L1 antibody and anlotinib or the pharmaceutically acceptable salt thereof described herein comprises a pharmaceutical composition of the anti-PD-L1 antibody, and a pharmaceutical composition of anlotinib or the pharmaceutically acceptable salt thereof, wherein the pharmaceutical composition of the anti-PD-L1 antibody is prepared in a unit dose or in multiple doses suitable for providing 600-2400 mg of the anti-PD-L1 antibody for a patient at first administration, and the pharmaceutical composition of anlotinib or the pharmaceutically acceptable salt thereof is prepared in a unit dose suitable for providing 6 mg, 8 mg, 10 mg, and/or 12 mg of anlotinib or the pharmaceutically acceptable salt thereof for a patient daily for 14 consecutive days.

In some embodiments, the pharmaceutical combination comprising the anti-PD-L1 antibody and anlotinib or the pharmaceutically acceptable salt thereof described herein comprises: a pharmaceutical composition in which the anti-PD-L1 antibody is at a concentration of 10-60 mg/mL, and a pharmaceutical composition comprising 6 mg, 8 mg, 10 mg, and/or 12 mg of anlotinib or the pharmaceutically acceptable salt thereof in a unit dose.

In some embodiments, the pharmaceutical combination comprising the anti-PD-L1 antibody and anlotinib or the pharmaceutically acceptable salt thereof described herein comprises: a pharmaceutical composition in which the anti-PD-L1 antibody is at a concentration of 30 mg/mL, and a pharmaceutical composition comprising 8 mg, 10 mg, and/or 12 mg of anlotinib or the pharmaceutically acceptable salt thereof in a unit dose.

In some embodiments, the pharmaceutical combination comprising the anti-PD-L1 antibody and anlotinib or the pharmaceutically acceptable salt thereof described herein comprises: a pharmaceutical composition in which the anti-PD-L1 antibody is at a concentration of 10 mg/mL, and a pharmaceutical composition comprising 8 mg, 10 mg, and/or 12 mg of anlotinib or the pharmaceutically acceptable salt thereof in a unit dose.

In some embodiments, the pharmaceutical combination comprising the anti-PD-L1 antibody and anlotinib or the pharmaceutically acceptable salt thereof described herein comprises: a pharmaceutical composition comprising 1200 mg of the anti-PD-L1 antibody provided in a multiple-dose form, and a pharmaceutical composition comprising 8 mg, 10 mg, and/or 12 mg of anlotinib or the pharmaceutically acceptable salt thereof in a unit dose. In some embodiments, the pharmaceutical combination comprising the anti-PD-L1 antibody and anlotinib or the pharmaceutically acceptable salt thereof described herein further comprises at least one third therapeutic agent, wherein the anti-PD-L1 antibody, anlotinib or the pharmaceutically acceptable salt thereof, and the third therapeutic agent are each in the form of a pharmaceutical composition and can be administered simultaneously, nonsimultaneously, or sequentially. In some embodiments, the third therapeutic agent is one or more of a fluorouracil anti-tumor drug, a platinum anti-tumor drug, and a paclitaxel anti-tumor drug. In some embodiments, the third therapeutic agent comprises capecitabine and/or a platinum anti-tumor drug. In some embodiments, the third therapeutic agent comprises a fluorouracil anti-tumor drug and/or oxaliplatin. In some embodiments, the third therapeutic agent comprises capecitabine and/or oxaliplatin. In some embodiments, the pharmaceutical combination comprising the anti-PD-L1 antibody and anlotinib or the pharmaceutically acceptable salt thereof further comprises a pharmaceutical composition of capecitabine and a pharmaceutical composition of oxaliplatin. In some embodiments, oxaliplatin is administered once every 3 weeks by intravenous drip. In some embodiments, capecitabine is administered orally on a regimen of consecutively 2-week treatment and then 1-week interruption. In some embodiments, oxaliplatin is administered once every 3 weeks by intravenous drip at a dose of 130 mg/m² each time. In some embodiments, capecitabine is administered orally at a single dose of 1000 mg/m² twice daily on a regimen of consecutively 2-week treatment and then 1-week interruption.

In some embodiments, the kit is suitable for administration within a single treatment cycle (e.g., a treatment cycle of 21 days), and comprises a pharmaceutical composition comprising 600-2400 mg of the anti-PD-L1 antibody and a pharmaceutical composition comprising 84-168 mg of anlotinib or the pharmaceutically acceptable salt thereof. In some embodiments, the kit further comprises a pharmaceutical composition of capecitabine and a pharmaceutical composition of oxaliplatin.

In some embodiments, provided is a method for treating gastric carcinoma and/or esophagogastric junction cancer, which comprises administering to a patient in need thereof a therapeutically effective amount of an anti-PD-L1 antibody, and anlotinib or a pharmaceutically acceptable salt thereof. In some embodiments, the anti-PD-L1 antibody, and anlotinib or the pharmaceutically acceptable salt thereof are administered simultaneously, nonsimultaneously, or sequentially. In some embodiments, the anti-PD-L1 antibody is administered once every 1 week, every 2 weeks, every 3 weeks, or every 4 weeks. In some embodiments, the anti-PD-L1 antibody is administered at a dose of 600-2400 mg each time. In some embodiments, the anti-PD-L1 antibody is administered at a single dose of 600 mg, 800 mg, 1000 mg, 1200 mg, 1400 mg, 1600 mg, 1800 mg, 2000 mg, 2200 mg, or 2400 mg. In some embodiments, anlotinib or the pharmaceutically acceptable salt thereof is administered at a daily dose of 6-12 mg. In some embodiments, anlotinib or the pharmaceutically acceptable salt thereof is administered at a daily dose of 8-12 mg. In some embodiments, anlotinib or the pharmaceutically acceptable salt thereof is administered at a daily dose of 6 mg, 8 mg, 10 mg, or 12 mg. In some embodiments, anlotinib or the pharmaceutically acceptable salt thereof is administered at a dose of 6 mg, 8 mg, 10 mg, or 12 mg once daily. In some embodiments, anlotinib or the pharmaceutically acceptable salt thereof is administered on a regimen of consecutively 2-week treatment and then 1-week interruption. In some embodiments, every 3 weeks is counted as one treatment cycle. In some embodiments, 21 days are counted as one treatment cycle, and the anti-PD-L1 antibody is administered to the patient on the first day of each treatment cycle. In some embodiments, every 3 weeks is counted as one treatment cycle, the anti-PD-L1 antibody is administered on the first day of each cycle, and anlotinib or the pharmaceutically acceptable salt thereof is administered on days 1-14 of each cycle. In some embodiments, the anti-PD-L1 antibody is administered parenterally. In some embodiments, the pharmaceutical composition of the anti-PD-L1 antibody is at a concentration of 10-60 mg/mL. In some embodiments, the pharmaceutical composition of the anti-PD-L1 antibody is at a concentration of 10 mg/mL, 20 mg/mL, 30 mg/mL, 40 mg/mL, 50 mg/mL, or 60 mg/mL. In some embodiments, anlotinib or the pharmaceutically acceptable salt thereof is administered orally.

In some embodiments, the method for treating gastric carcinoma and/or esophagogastric junction cancer further comprises administering to a patient in need thereof a therapeutically effective amount of at least one third therapeutic agent. In some embodiments, the third therapeutic agent is one or more of a fluorouracil anti-tumor drug, a platinum anti-tumor drug, and a paclitaxel anti-tumor drug. In some embodiments, the third therapeutic agent comprises capecitabine and/or a platinum anti-tumor drug. In some embodiments, the third therapeutic agent comprises a fluorouracil anti-tumor drug and/or oxaliplatin. In some embodiments, the third therapeutic agent comprises capecitabine and/or oxaliplatin. In some embodiments, the method comprises administering oxaliplatin at a dose of 130 mg/m² each time and administering capecitabine at a dose of 1000 mg/m² each time. In some embodiments, the anti-PD-L1 antibody, anlotinib or the pharmaceutically acceptable salt thereof, oxaliplatin, and capecitabine are administered simultaneously, nonsimultaneously, or sequentially. In some embodiments, the anti-PD-L1 antibody, anlotinib, oxaliplatin, and capecitabine have the same treatment cycle, e.g., a 1-week, 2-week, 3-week or 4-week treatment cycle. In some embodiments, every 3 weeks is counted as one treatment cycle, the anti-PD-L1 antibody and oxaliplatin are administered on the first day of each cycle, and anlotinib or the pharmaceutically acceptable salt thereof and capecitabine are administered on days 1-14 of each cycle. In some embodiments, the method comprises: administering the anti-PD-L1 antibody, and anlotinib or the pharmaceutically acceptable salt thereof, and the third therapeutic agent for an initial treatment; and then optionally administering the anti-PD-L1 antibody, and anlotinib or the pharmaceutically acceptable salt thereof for a maintenance treatment. In some embodiments, the method comprises: with every 3 weeks counted as one treatment cycle, administering the anti-PD-L1 antibody and oxaliplatin on the first day of each cycle and administering anlotinib or the pharmaceutically acceptable salt thereof and capecitabine on days 1-14 of each cycle for the initial treatment; and then optionally, with every 3 weeks counted as one treatment cycle, administering the anti-PD-L1 antibody on the first day of each cycle and administering anlotinib or the pharmaceutically acceptable salt thereof on days 1-14 of each cycle for the maintenance treatment. In some embodiments, the initial treatment comprises 0 to 10 treatment cycles, preferably 1 to 10 treatment cycles, more preferably 1 to 6 treatment cycles, and most preferably 6 treatment cycles. In some embodiments, the initial treatment comprises 0 to 10 consecutive treatment cycles, preferably 1 to 10 consecutive treatment cycles, more preferably 1 to 6 consecutive treatment cycles, and most preferably 6 consecutive treatment cycles, from the first dose.

In yet another aspect, the present application provides a method for treating gastric carcinoma and/or esophagogastric junction cancer, which comprises: (1) obtaining or having obtained a biological sample from a patient; (2) testing or having tested the biological sample; and (3) determining whether the sample is HER2/neu-negative (or has indeterminate HER2/neu status), and if the sample is tested to be HER2/neu-negative (or have indeterminate HER2/neu status), administering to the patient a therapeutically effective amount of an anti-PD-L1 antibody, and anlotinib or a pharmaceutically acceptable salt thereof; and preferably, in some embodiments, further administering to the patient a therapeutically effective amount of a third therapeutic agent; or (3') determining whether the sample is HER2/neu-negative (or has indeterminate HER2/neu status), and if the sample is tested to be HER2/neu-negative (or have indeterminate HER2/neu status), administering to the patient a therapeutically effective amount of an anti-PD-L1 antibody, and anlotinib or a pharmaceutically acceptable salt thereof, and a third therapeutic agent for an initial treatment; and then optionally administering the anti-PD-L1 antibody, and anlotinib or the pharmaceutically acceptable salt thereof for a maintenance treatment until the patient is intolerant or the disease progresses. In some embodiments, the initial treatment comprises 0 to 10 treatment cycles, preferably 1 to 10 treatment cycles, more preferably 1 to 6 treatment cycles, and most preferably 6 treatment cycles. In some embodiments, the initial treatment comprises 0 to 10 consecutive treatment cycles, preferably 1 to 10 consecutive treatment cycles, more preferably 1 to 6 consecutive treatment cycles, and most preferably 6 consecutive treatment cycles, from the first dose. In some embodiments, the third therapeutic agent is one or more of a fluorouracil anti-tumor drug and/or a platinum anti-tumor drug and/or a paclitaxel anti-tumor drug. In some embodiments, the third therapeutic agent comprises a fluorouracil anti-tumor drug and/or a platinum anti-tumor drug. In some embodiments, the third therapeutic agent comprises capecitabine and/or a platinum anti-tumor drug. In some embodiments, the third therapeutic agent comprises a fluorouracil anti-tumor drug and/or oxaliplatin. In some embodiments, the third therapeutic agent comprises capecitabine and/or oxaliplatin.

### Anlotinib or a pharmaceutically acceptable salt thereof

The chemical name of anlotinib is 1-[[[4-(4-fluoro-2-methyl-1*H*-indol-5-yl)oxy-6-methoxyquinolin-7-yl]oxy] methyl]cyclopropylamine, which has the following structural formula:

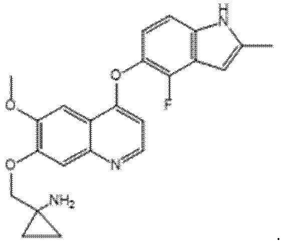

The pharmaceutically acceptable salt includes, but is not limited to, salts formed from anlotinib and acids selected from the group consisting of the following: hydrochloric acid, hydrobromic acid, sulfuric acid, nitric acid, phosphoric acid, acetic acid, trifluoroacetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, *p*-chlorobenzenesulfonic acid, *p*-toluenesulfonic acid, 3-phenylpropionic acid, trimethylacetic acid, *t*-butylacetic acid, dodecyl sulfuric acid, gluconic acid, glutamic acid, hydroxyl naphthoic acid, salicylic acid, and stearic acid. In some embodiments, the pharmaceutically acceptable salt is a hydrochloride or a maleate; in some embodiments, the pharmaceutically acceptable salt is a dihydrochloride.

Unless otherwise stated, the dose of anlotinib or the pharmaceutically acceptable salt thereof referred to in the present application is based on the molecular weight of the free base of anlotinib.

Anlotinib or the pharmaceutically acceptable salt thereof may be administered through various routes including gastrointestinal and parenteral including, but not limited to, oral, intravenous, intra-arterial, transdermal, sublingual, intramuscular, rectal, transbuccal, intranasal, inhalational, vaginal, intraocular, subcutaneous, intra-adipose, intra-articular, intraperitoneal, and intrathecal administrations. In some specific embodiments, anlotinib or the pharmaceutically acceptable salt thereof is administered orally. The amount of anlotinib or the pharmaceutically acceptable salt thereof administered can be determined according to the severity of the disease, the response of the disease, any treatment-related toxicity, and the age and health of a patient. For example, anlotinib or the pharmaceutically acceptable salt thereof can be administered at a daily dose of 2 mg to 20 mg; in some embodiments, anlotinib or the pharmaceutically acceptable salt thereof can be administered at a daily dose of 2 mg, 3 mg, 4 mg, 5 mg, 6 mg, 7 mg, 8 mg, 9 mg, 10 mg, 11 mg, 12 mg, 13 mg, 14 mg, 15 mg, or 16 mg. Anlotinib or the pharmaceutically acceptable salt thereof can be administered once or multiple times daily. In some embodiments, anlotinib or the pharmaceutically acceptable salt thereof is administered once daily in the form of an oral solid formulation.

The administration regimen for anlotinib or the pharmaceutically acceptable salt thereof can be determined comprehensively depending on the activity and toxicity of the drug, the tolerance of a patient, and the like. Preferably, anlotinib or the pharmaceutically acceptable salt thereof is administered at intervals. The interval administration comprises a treatment period and an interruption period. Anlotinib or the pharmaceutically acceptable salt thereof can be administered once or multiple times daily in the treatment period. For example, the ratio of the treatment period to the interruption period in days is 2:(0.5-5), 2:(0.5-3), 2:(0.5-2), or 2:(0.5-1). In some embodiments, anlotinib or the pharmaceutically acceptable salt thereof is administered on a regimen of consecutively 2-week treatment and then 2-week interruption. In some embodiments, anlotinib or the pharmaceutically acceptable salt thereof is administered on a regimen of consecutively 2-week treatment and then 1-week interruption. In some embodiments, anlotinib or the pharmaceutically acceptable salt thereof is administered on a regimen of consecutively 5-day treatment and then 2-day interruption. For example, anlotinib or the pharmaceutically acceptable salt thereof can be administered orally at a dose of 6 mg, 8 mg, 10 mg, or 12 mg once daily on a regimen of consecutively 2-week treatment and then 1-week interruption.

### Pharmaceutical composition of anlotinib or the pharmaceutically acceptable salt thereof

In some embodiments of the present application, a unit dose of the pharmaceutical composition of anlotinib or the pharmaceutically acceptable salt thereof comprises 6 mg, 8 mg, 10 mg, or 12 mg of anlotinib.

In some embodiments of the present application, according to a treatment cycle of 2-week treatment and then 1-week interruption, a total dose of the pharmaceutical composition of anlotinib or the pharmaceutically acceptable salt thereof administered per cycle includes 84-168 mg. In some embodiments, a total dose of the pharmaceutical composition of anlotinib or the pharmaceutically acceptable salt thereof includes an amount selected from the group consisting of 84 mg, 112 mg, 140 mg and 168 mg or from a range formed of any of the aforementioned values. In some embodiments, a total dose of the pharmaceutical composition of anlotinib or the pharmaceutically acceptable salt thereof includes 112-168 mg.

In some embodiments, the pharmaceutical composition includes, but is not limited to, formulations suitable for oral, parenteral and local administrations. In some embodiments, the pharmaceutical composition is a formulation suitable for oral administration. In some embodiments, the pharmaceutical composition is a solid formulation suitable for oral administration. In some embodiments, the pharmaceutical composition includes, but is not limited to, a tablet and a capsule.

### Oxaliplatin

The chemical name of oxaliplatin is (1*R*-*trans*)-(1,2-cyclohexanediamine-*N*,*N*')[oxalic acid (2-)-O,O']platinum, which has the following structural formula:

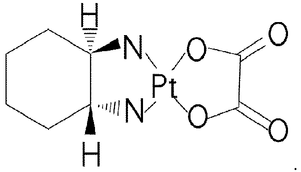

Oxaliplatin may be administered through various routes including gastrointestinal and parenteral including, but not limited to, oral, intravenous, intra-arterial, transdermal, sublingual, intramuscular, rectal, transbuccal, intranasal, inhalational, vaginal, intraocular, subcutaneous, intra-adipose, intra-articular, intraperitoneal, and intrathecal administrations. In some specific embodiments, oxaliplatin is administered by injection.

In the present application, oxaliplatin may be administered at a single dose of 65-130 mg/m²; in some embodiments, oxaliplatin may be administered at a single dose of 85-130 mg/m². In some embodiments, oxaliplatin is administered as an injection once every 2 weeks. In some embodiments, oxaliplatin is administered as an injection once every 3 weeks, for example, oxaliplatin is administered by injection at a dose of 130 mg/m² on the first day of each cycle. It will be understood, based on the general knowledge of those skilled in the art, that mg/m² refers to the dose of a drug used per square meter of body surface area of a subject.

### Pharmaceutical composition of oxaliplatin

In some embodiments of the present application, the pharmaceutical composition of oxaliplatin includes, but is not limited to, formulations suitable for intravenous, oral, parenteral and local administrations; in some embodiments, the pharmaceutical composition is a formulation suitable for injection; in some embodiments, the pharmaceutical composition includes, but is not limited to, a lyophilized powder injection.

### Capecitabine

The chemical name of capecitabine is pentyl [1-(5-deoxy-β-D-ribofuranosyl)-5-fluoro-2-oxo-1,2-dihydropyrimidin-4-yl]carbamate, which has the following structural formula:

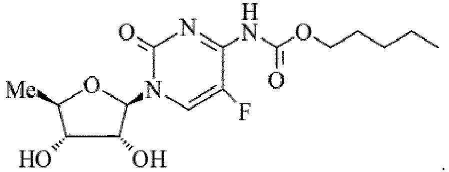

Capecitabine may be administered through various routes including gastrointestinal and parenteral including, but not limited to, oral, intravenous, intra-arterial, transdermal, sublingual, intramuscular, rectal, transbuccal, intranasal, inhalational, vaginal, intraocular, subcutaneous, intra-adipose, intra-articular, intraperitoneal, and intrathecal administrations. In some specific embodiments, capecitabine is administered orally.

In the present application, capecitabine may be administered at a single dose of 1000-1250 mg/m²; in some embodiments, capecitabine may be administered at a single dose of 1000 mg/m². In some embodiments, capecitabine is administered at a single dose of 1000 mg/m² twice daily on a regimen of consecutively 2-week treatment and then 1-week interruption. It will be understood, based on the general knowledge of those skilled in the art, that mg/m² refers to the dose of a drug used per square meter of body surface area of a subject.

### Pharmaceutical composition of capecitabine

In some embodiments of the present application, the pharmaceutical composition of capecitabine includes, but is not limited to, formulations suitable for intravenous, oral, parenteral and local administrations; in some embodiments, the pharmaceutical composition is a formulation suitable for oral administration; in some embodiments, the pharmaceutical composition includes, but is not limited to, a tablet.

### Anti-PD-L1 antibody

In some embodiments of the present application, the anti-PD-L1 antibody is the antibody disclosed in WO2016022630 or CN107001463A.

In some embodiments of the present application, the anti-PD-L1 antibody comprises the following amino acid sequences: a heavy chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 1 or SEQ ID NO: 4; a heavy chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 2 or SEQ ID NO: 5; a heavy chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 3 or SEQ ID NO: 6; a light chain CDR1 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 7 or SEQ ID NO: 10; a light chain CDR2 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 8 or SEQ ID NO: 11; and a light chain CDR3 region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 9 or SEQ ID NO: 12.

In some embodiments of the present application, the anti-PD-L1 antibody comprises the following amino acid sequences: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12.

In some embodiments of the present application, the isolated anti-PD-L1 antibody described herein comprises: a heavy chain CDR1 region having an amino acid sequence set forth in SEQ ID NO: 1; a heavy chain CDR2 region having an amino acid sequence set forth in SEQ ID NO: 2; a heavy chain CDR3 region having an amino acid sequence set forth in SEQ ID NO: 3; a light chain CDR1 region having an amino acid sequence set forth in SEQ ID NO: 7; a light chain CDR2 region having an amino acid sequence set forth in SEQ ID NO: 8; and a light chain CDR3 region having an amino acid sequence set forth in SEQ ID NO: 9.

Each of the CDR regions described herein and the variants thereof described above are capable of specifically recognizing and binding to PD-L1, thereby effectively blocking the signaling between PD-L1 and PD-1.

In some embodiments of the present application, the anti-PD-L1 antibody comprises the following amino acid sequences: a heavy chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 13 or SEQ ID NO: 14; and a light chain variable region having at least 80% (e.g., 81%, 82%, 83%, 84%, 85%, 86%, 87%, 88%, 89%, 90%, 91%, 92%, 93%, 94%, 95%, 96%, 97%, 98%, 99%, or 100%) homology to an amino acid sequence set forth in SEQ ID NO: 15 or SEQ ID NO: 16.

In some embodiments of the present application, the anti-PD-L1 antibody comprises the following amino acid sequences: a heavy chain variable region set forth in SEQ ID NO: 13, and a light chain variable region set forth in SEQ ID NO: 15.

In some embodiments of the present application, the anti-PD-L1 antibody comprises the following amino acid sequences: a heavy chain variable region set forth in SEQ ID NO: 14, and a light chain variable region set forth in SEQ ID NO: 16.

In some embodiments of the present application, the anti-PD-L1 antibody comprises the following amino acid sequences: a heavy chain amino acid sequence set forth in SEQ ID NO: 17, and a light chain amino acid sequence set forth in SEQ ID NO: 18.

In some embodiments of the present application, the anti-PD-L1 antibody comprises the following amino acid sequences: a heavy chain amino acid sequence set forth in SEQ ID NO: 19, and a light chain amino acid sequence set forth in SEQ ID NO: 20.

In some embodiments of the present application, the anti-PD-L1 antibody comprises the following amino acid sequences: a heavy chain amino acid sequence set forth in SEQ ID NO: 21, and a light chain amino acid sequence set forth in SEQ ID NO: 18.

In one specific embodiment, the humanized anti-PD-L1 mAb disclosed herein comprises one or more conservatively substituted variants selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, and SEQ ID NO: 21. The humanized anti-PD-L1 mAb comprising the conservatively substituted variants retains the ability to specifically recognize and bind to PD-L1.

In some embodiments of the present application, the anti-PD-L1 antibody may be an IgG1 or IgG4 antibody.

In some embodiments of the present application, the anti-PD-L1 antibody is an IgG1 antibody. In some embodiments, the anti-PD-L1 antibody is a glycosylated IgG1 antibody.

In some embodiments of the present application, the anti-PD-L1 antibody comprises heavy chain complementarity determining regions (CDRs) selected from the group consisting of heavy chain CDRs derived from antibodies 13C5 and 5G11, and light chain CDRs selected from the group consisting of light chain CDRs derived from antibodies 13C5 and 5G11. In one embodiment, the anti-PD-L1 antibody described herein comprises: a heavy chain variable region of chimeric antibodies selected from the group consisting of ch5G11-hIgGl, ch5G11-hIgG4, ch13C5-hIgG 1 and ch13C5-hIgG4; and a light chain variable region of chimeric antibodies selected from the group consisting of ch5G11-hIgG1, ch5G11-hIgG4, ch13C5-hIgG1 and ch13C5-hIgG4. In one embodiment, the anti-PD-L1 antibody described herein comprises: a heavy chain variable region of humanized antibodies selected from the group consisting of hu13C5-hIgG1, hu13C5-hIgG4, hu5G11-hIgG1 and hu5G11-hIgG4; and a light chain variable region of humanized antibodies selected from the group consisting of hu13C5-hIgG1, hu13C5-hIgG4, hu5G11-hIgG1 and hu5G11-hIgG4. Reference can be made to the description of the patent WO2016022630 or CN107001463A: 13C5, ch13C5-hIgG1, ch13C5-hIgG4, hu13C5-hIgG1, or hu13C5-hIgG4 comprises an HCDR1 sequence of SYGMS (SEQ ID NO: 4), an HCDR2 sequence of SISSGGSTYYPDSVKG (SEQ ID NO: 5), an HCDR3 sequence of GYDSGFAY (SEQ ID NO: 6), an LCDR1 sequence of ASQSVSTSSSSFMH (SEQ ID NO: 10), an LCDR2 sequence of YASNLES (SEQ ID NO: 11), and an LCDR3 sequence of QHSWEIPYT (SEQ ID NO: 12); and 5G11, ch5G11-hIgG1, ch5G11-hIgG4, hu5G11-hIgG1, or hu5G11-hIgG4 comprises an HCDR1 sequence of TYGVH (SEQ ID NO: 1), an HCDR2 sequence of VIWRGVTTDYNAAFMS (SEQ ID NO: 2), an HCDR3 sequence of LGFYAMDY (SEQ ID NO: 3), an LCDR1 sequence of KASQSVSNDVA (SEQ ID NO: 7), an LCDR2 sequence of YAANRYT (SEQ ID NO: 8), and an LCDR3 sequence of QQDYTSPYT (SEQ ID NO: 9).

In some embodiments of the present application, the anti-PD-L1 antibody in the pharmaceutical combination may be selected from one or more. As used herein, the term "more" refers to more than one, for example, two, three, four, five, or more. For example, in some embodiments of the present application, the anti-PD-L1 antibody is selected from the group consisting of an antibody comprising a heavy chain variable region set forth in SEQ ID NO: 13 and a light chain variable region set forth in SEQ ID NO: 15, or selected from the group consisting of an antibody comprising a heavy chain variable region set forth in SEQ ID NO: 14 and a light chain variable region set forth in SEQ ID NO: 16, or selected from the group consisting of a combination thereof. As another example, the anti-PD-L1 antibody is selected from the group consisting of an antibody comprising a heavy chain amino acid sequence set forth in SEQ ID NO: 17 and a light chain amino acid sequence set forth in SEQ ID NO: 18, or selected from the group consisting of an antibody comprising a heavy chain amino acid sequence set forth in SEQ ID NO: 19 and a light chain amino acid sequence set forth in SEQ ID NO: 20, or selected from the group consisting of an antibody comprising a heavy chain amino acid sequence set forth in SEQ ID NO: 21 and a light chain amino acid sequence set forth in SEQ ID NO: 18, or selected from the group consisting of combinations of any of the foregoing.

In some embodiments, the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

### Pharmaceutical composition of anti-PD-L1 antibody

In some embodiments of the present application, the pharmaceutical composition of the anti-PD-L1 antibody comprises 600-2400 mg of the anti-PD-L1 antibody. In some embodiments, the pharmaceutical composition of the anti-PD-L1 antibody comprises the anti-PD-L1 antibody in an amount selected from the group consisting of 600 mg, 900 mg, 1200 mg, 1500 mg, 1800 mg, 2100 mg and 2400 mg or from a range formed of any of the aforementioned values. In some embodiments, the pharmaceutical composition of the anti-PD-L1 antibody comprises 600-2100 mg or 900-1500 mg of the anti-PD-L1 antibody, wherein the pharmaceutical composition of the anti-PD-L1 antibody may be present in multiple-dose or unit-dose form.

In some embodiments of the present application, the pharmaceutical composition of the anti-PD-L1 antibody comprises 300 mg, 600 mg, or 1200 mg of the anti-PD-L1 antibody. In some embodiments of the present application, provided is a pharmaceutical composition of an anti-PD-L1 antibody formulated as a unit dose, which comprises 300 mg, 600 mg, or 1200 mg of the anti-PD-L1 antibody.

In some embodiments, the pharmaceutical composition of the anti-PD-L1 antibody is a solution for injection. In some embodiments, the pharmaceutical composition of the anti-PD-L1 antibody is an aqueous solution for injection. In some embodiments of the present application, the pharmaceutical composition of the anti-PD-L1 antibody comprises one or more of a buffer, an isotonicity modifier, a stabilizer, and/or a surfactant. In particular, the pharmaceutical composition of the anti-PD-L1 antibody comprises 1-150 mg/mL anti-PD-L1 antibody (e.g., mAb), 3-50 mM buffer, 2-150 mg/mL isotonicity modifier/stabilizer, and 0.01-0.8 mg/mL surfactant, and has a pH of 4.5-6.8.

In some embodiments of the present application, in the pharmaceutical composition of the anti-PD-L1 antibody, the anti-PD-L1 mAb is at a concentration of 5-150 mg/mL (w/v); in some embodiments, the concentration is 10-60 mg/mL (w/v); in some embodiments, the concentration is 10-30 mg/mL (w/v). In some specific embodiments, the anti-PD-L1 mAb is at a concentration of 10 mg/mL, 20 mg/mL, 30 mg/mL, 40 mg/mL, 50 mg/mL, 60 mg/mL, 70 mg/mL, 80 mg/mL, 90 mg/mL, 100 mg/mL, 110 mg/mL, or 120 mg/mL (w/v); in some embodiments, the concentration is 10 mg/mL, 20 mg/mL, 30 mg/mL, 40 mg/mL, 50 mg/mL, or 60 mg/mL (w/v); in some embodiments, the concentration is 10 mg/mL, 20 mg/mL, or 30 mg/mL (w/v). In some embodiments, the anti-PD-L1 mAb is at a concentration of 10 mg/mL (w/v). In other embodiments, the anti-PD-L1 mAb is at a concentration of 30 mg/mL (w/v). In other embodiments, the anti-PD-L1 mAb is at a concentration of 60 mg/mL (w/v).

In some embodiments of the present application, the buffer is a histidine salt buffer. The histidine salt buffer is at a concentration of 5-30 mM; in some embodiments, the concentration is 10-25 mM; in some embodiments, the concentration is 10-20 mM; in some embodiments, the concentration is 10-15 mM. In some specific embodiments, the histidine salt buffer is at a concentration of 5 mM, 10 mM, 15 mM, 20 mM, 25 mM, or 30 mM. In some embodiments, the histidine salt buffer is at a concentration of 10 mM. In other embodiments, the histidine salt buffer is at a concentration of 15 mM. In other embodiments, the histidine salt buffer is at a concentration of 20 mM. The histidine salt buffer comprises histidine and hydrochloric acid.

In some embodiments of the present application, the isotonicity modifier/stabilizer is sucrose at 20-150 mg/mL (w/v); in some embodiments, the isotonicity modifier/stabilizer is sucrose at 40-100 mg/mL (w/v); in some embodiments, the isotonicity modifier/stabilizer is sucrose at 60-80 mg/mL (w/v). In some specific embodiments, the sucrose is at a concentration of 40 mg/mL, 50 mg/mL, 60 mg/mL, 70 mg/mL, 80 mg/mL, 90 mg/mL, or 100 mg/mL. In some specific embodiments, the sucrose is at a concentration of 60 mg/mL. In some specific embodiments, the sucrose is at a concentration of 70 mg/mL. In some specific embodiments, the sucrose is at a concentration of 80 mg/mL. In some specific embodiments, the sucrose is at a concentration of 90 mg/mL.

In some embodiments of the present application, the surfactant is selected from the group consisting of polysorbate 80, polysorbate 20, and poloxamer 188; in some embodiments, the surfactant is selected from the group consisting of polysorbate 80 and polysorbate 20; in some embodiments, the surfactant is selected from polysorbate 80. In some embodiments, the surfactant is at a concentration of 0.05-0.6 mg/mL (w/v); in some embodiments, the concentration is 0.1-0.4 mg/mL (w/v); in some embodiments, the concentration is 0.2-0.3 mg/mL (w/v).

In some embodiments of the present application, the surfactant is polysorbate 80 or polysorbate 20 at 0.01-0.8 mg/mL (w/v). In some specific embodiments, the surfactant is polysorbate 80 at 0.05-0.6 mg/mL; in some embodiments, the surfactant is polysorbate 80 at 0.1-0.4 mg/mL; in some embodiments, the surfactant is polysorbate 80 at 0.2-0.3 mg/mL; in some embodiments, the surfactant is polysorbate 80 at 0.2 mg/mL. In some embodiments, the pharmaceutical composition has a polysorbate 80 content of 0.1 mg/mL, 0.2 mg/mL, 0.3 mg/mL, 0.4 mg/mL, 0.5 mg/mL, or 0.6 mg/mL; in some embodiments, the pharmaceutical composition has a polysorbate 80 content of 0.2 mg/mL, 0.3 mg/mL, 0.4 mg/mL, or 0.5 mg/mL; in some embodiments, the pharmaceutical composition has a polysorbate 80 content of 0.2 mg/mL, 0.3 mg/mL, or 0.4 mg/mL; in some embodiments, the pharmaceutical composition has a polysorbate 80 content of 0.2 mg/mL. In some embodiments, the pharmaceutical composition has a polysorbate 80 content of 0.1 mg/mL. In other embodiments, the pharmaceutical composition has a polysorbate 80 content of 0.2 mg/mL. In some embodiments, the pharmaceutical composition has a polysorbate 80 content of 0.3 mg/mL. In other embodiments, the pharmaceutical composition has a polysorbate 80 content of 0.4 mg/mL. In some embodiments, the pharmaceutical composition has a polysorbate 80 content of 0.5 mg/mL.

In some embodiments of the present application, an aqueous solution of the pharmaceutical composition has a pH selected from 4.0-6.8; in some embodiments, the pH is 4.5-6.5; in some embodiments, the pH is 5.5-6.0; in some embodiments, the pH is 5.5. In some embodiments, an aqueous solution of the pharmaceutical composition has a pH of 4.5, 4.8, 5.0, 5.2, 5.4, 5.5, 5.6, 5.8, or 6.0; in some embodiments, the pH is 5.0, 5.2, 5.4, 5.5, or 5.6; in some embodiments, the pH is 5.5. In some embodiments, an aqueous solution of the pharmaceutical composition has a pH of 5.0. In some embodiments, an aqueous solution of the pharmaceutical composition has a pH of 5.2. In some embodiments, an aqueous solution of the pharmaceutical composition has a pH of 5.4. In some embodiments, an aqueous solution of the pharmaceutical composition has a pH of 5.5. In some embodiments, an aqueous solution of the pharmaceutical composition has a pH of 5.6. In some embodiments, an aqueous solution of the pharmaceutical composition has a pH of 5.8. In some embodiments, an aqueous solution of the pharmaceutical composition has a pH of 6.0.

In some specific embodiments of the present application, the pharmaceutical composition comprises: (a) an anti-PD-L1 antibody at a concentration of 20 mg/mL (w/v), (b) sucrose at a concentration of 70 mg/mL (w/v), (c) polysorbate 80 at a concentration of 0.1 mg/mL (w/v), (d) histidine at a concentration of 20 mM, and (e) optionally a suitable amount of hydrochloric acid for adjusting the pH of the composition to 5.0. In one specific embodiment of the present application, the pharmaceutical composition comprises: (a) an anti-PD-L1 mAb at a concentration of 20 mg/mL (w/v), (b) sucrose at a concentration of 70 mg/mL (w/v), (c) polysorbate 80 at a concentration of 0.1 mg/mL (w/v), (d) histidine at a concentration of 20 mM, and (e) optionally a suitable amount of hydrochloric acid for adjusting the pH of the composition to 5.0.

In another specific embodiment of the present application, the pharmaceutical composition comprises: (a) an anti-PD-L1 antibody at a concentration of 10 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 50 mg/mL (w/v), (b) sucrose at a concentration of 80 mg/mL (w/v), (c) polysorbate 80 at a concentration of 0.3 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 100 mg/mL (w/v), (b) sucrose at a concentration of 80 mg/mL (w/v), (c) polysorbate 80 at a concentration of 0.5 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 30 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 60 mg/mL (w/v), (b) sucrose at a concentration of 80 mg/mL (w/v), (c) polysorbate 80 at a concentration of 0.2 mg/mL (w/v), (d) histidine at a concentration of 10 mM, and (e) optionally a suitable amount of hydrochloric acid for adjusting the pH of the composition to 5.5.

In another specific embodiment of the present application, the pharmaceutical composition comprises: (a) an anti-PD-L1 antibody at a concentration of 10 mg/mL (w/v), (b) sucrose at a concentration of 70 mg/mL (w/v), (c) polysorbate 80 at a concentration of 0.4 mg/mL (w/v), (d) histidine at a concentration of 20 mM, and (e) optionally a suitable amount of acetic acid for adjusting the pH of the composition to 6.5.

In another specific embodiment of the present application, the pharmaceutical composition comprises: (a) an anti-PD-L1 mAb at a concentration of 10 mg/mL (w/v), (b) sucrose at a concentration of 80 mg/mL (w/v), (c) polysorbate 80 at a concentration of 0.2 mg/mL (w/v), (d) histidine at a concentration of 20 mM, and (e) optionally a suitable amount of hydrochloric acid for adjusting the pH of the composition to 5.5.

In another specific embodiment of the present application, the pharmaceutical composition is a water-soluble injection; in some embodiments, the water-soluble injection includes, but is not limited to, a water-soluble formulation without lyophilization or a water-soluble formulation reconstituted from a lyophilized powder. In other embodiments, the pharmaceutical composition is a lyophilized formulation. The lyophilized formulation refers to a formulation prepared by subjecting an aqueous solution to a lyophilization process in which a substance is first frozen, and then the amount of a solvent is reduced by sublimation (primary drying process) and then by desorption (secondary drying process) until the amount of the solvent is reduced to a value that no longer supports a biological activity or a chemical reaction. The lyophilized formulation of the present application can also be dried by other methods known in the art, such as spray drying and bubble drying.

### Gastric carcinoma

In the present application, the gastric carcinoma includes, but is not limited to, adenocarcinoma, adenosquamous carcinoma, squamous cell carcinoma, poorly cohesive carcinoma, or anaplastic carcinoma.

In some embodiments of the present application, the gastric carcinoma includes, but is not limited to, tubular adenocarcinoma, parietal cell adenocarcinoma, mixed adenocarcinoma, papillary adenocarcinoma, mucoepidermoid carcinoma, mucinous adenocarcinoma, signet-ring cell carcinoma, poorly cohesive carcinoma, hepatoid adenocarcinoma, and paneth cell carcinoma.

In some embodiments of the present application, the gastric carcinoma is non-HER2-positive gastric carcinoma. In some embodiments, the non-HER2-positive gastric carcinoma includes gastric carcinoma that is HER2 negative or has indeterminate HER2 status.

In some embodiments of the present application, the gastric carcinoma is advanced gastric carcinoma. In some embodiments of the present application, the gastric carcinoma is refractory gastric carcinoma.

In some embodiments of the present application, the gastric carcinoma is recurrent and/or metastatic gastric carcinoma.

In some embodiments of the present application, the gastric carcinoma is recurrent and/or metastatic advanced gastric carcinoma.

In some embodiments of the present application, the gastric carcinoma is unresectable locally advanced or metastatic gastric carcinoma.

In some embodiments of the present application, the patient with gastric carcinoma has not previously received drug therapy, or has previously received drug therapy but has failed or was intolerant to the therapy.

In some embodiments of the present application, the drug therapy includes chemotherapy, targeted drug therapy, and immunotherapy.

In some embodiments of the present application, the drug used for the chemotherapy includes, but is not limited to, one or more of taxanes, vinca alkaloids, platinums, fluorouracils, camptothecin analogs, anthracycline compounds, and podophyllums. The taxanes include, but are not limited to, one or more of paclitaxel, paclitaxel liposome, albumin-bound paclitaxel, and docetaxel; the vinca alkaloids include, but are not limited to, one or more selected from the group consisting of vinblastine, vincristine, vindesine, vinorelbine, vinflunine, and navelbine; the platinums include, but are not limited to, one or more selected from the group consisting of miriplatin, cisplatin, carboplatin, dicycloplatin, nedaplatin, oxaliplatin, lobaplatin, triplatin tetranitrate, phenanthriplatin, picoplatin, and satraplatin; the fluorouracils include, but are not limited to, one or more selected from the group consisting of cytarabine, azacitidine, ancitabine, capecitabine, gemcitabine, 5-fluorouracil, difuradin, doxifluridine, trifluridine, tegafur, carmofur, tegafur-gimeracil-oteracil potassium, and uracil-tegafur; the camptothecin analogs include, but are not limited to, one or more of camptothecin, hydroxycamptothecin, irinotecan, and topotecan; the anthracyclines include, but are not limited to, one or more of epirubicin, adriamycin, daunorubicin, pirarubicin, amrubicin, idarubicin, mitoxantrone, aclarubicin, valrubicin, zorubicin, pixantrone, pirarubicin, and liposomal doxorubicin; the podophyllums include, but are not limited to, one or more of etoposide, teniposide, and etoposide.

In some embodiments of the present application, the targeted drug includes, but is not limited to, one or more of an EGFR antagonist, a VEGF inhibitor, an HER2 inhibitor, a PARP inhibitor, a PI3K/Akt/mTOR pathway inhibitor, an epigenetic modification inhibitor, an HDAC inhibitor, or an FGFR inhibitor.

In some embodiments of the present application, the EGFR antagonist includes, but is not limited to, one or more of trastuzumab, cetuximab, icotinib, gefitinib, erlotinib, and lapatinib. In some embodiments of the present application, the HER2 inhibitor includes, but is not limited to, trastuzumab, inetetamab, pertuzumab, T-DM1, Enhertu, pyrotinib, neratinib, tucatinib, Phesgo, and Margenza. In some embodiments of the present application, the VEGF inhibitor includes, but is not limited to, bevacizumab, ranibizumab, axitinib, motesanib, aflibercept, cediranib, nintedanib, sorafenib, apatinib, or sunitinib. In some embodiments of the present application, the PARP inhibitor includes, but is not limited to, one or more of olaparib, niraparib, or rucaparib. In some embodiments of the present application, the PI3K/Akt/mTOR pathway inhibitor includes, but is not limited to, one or more of NVP-BKM120, XL147, perifosine, rapamycin, temsirolimus, everolimus, sirolimus, or ridaforolimus. In some embodiments of the present application, the epigenetic modification inhibitor includes, but is not limited to, one or more of azacitidine, decitabine, zebularine, fazarabine, romidepsin, vorinostat, belinostat, or chidamide. In some embodiments of the present application, the HDAC inhibitor includes, but is not limited to, one or more of trichostatin A, suberoylanilidehydroxamic acid, or sodiumbutyrate (NaB). In some embodiments of the present application, the FGFR inhibitor includes, but is not limited to, dovitinib or NVP-BGJ398.

In some embodiments of the present application, the drug for the immunotherapy includes, but is not limited to, one or more of a PD-1/PD-L1 inhibitor, a CTLA-4 inhibitor, a LAG-3 inhibitor, and a TIM-3 inhibitor. In some embodiments of the present application, the PD-1/PD-L1 inhibitor includes, but is not limited to, one or more of atezolizumab, nivolumab, pembrolizumab, durvalumab, avelumab, and cemiplimab. In some embodiments of the present application, the CTLA-4 inhibitor includes, but is not limited to, one or more of ipilimumab, tremelimumab, AGEN1884, and KN046. In some embodiments of the present application, the LAG-3 inhibitor includes, but is not limited to, one or more of relatlimab, fianlimab, IMP321, BMS-986016, and GSK 2831781. In some embodiments of the present application, the TIM-3 inhibitor includes, but is not limited to, one or more of MBG453, TSR-022, BMS-986258, RO7121661, and LY3321367.

### Esophagogastric junction cancer

In some embodiments of the present application, the esophagogastric junction cancer includes adenocarcinoma of esophagogastric junction (AEG).

In some embodiments of the present application, the esophagogastric junction cancer includes non-HER2-positive esophagogastric junction cancer. In some embodiments, the esophagogastric junction cancer is non-HER2-positive adenocarcinoma of esophagogastric junction. In some embodiments, the non-HER2-positive includes HER2-negative or indeterminate HER2 status.

In some embodiments of the present application, the esophagogastric junction cancer is advanced esophagogastric junction cancer. In some embodiments of the present application, the esophagogastric junction cancer is refractory esophagogastric junction cancer. In some embodiments of the present application, the esophagogastric junction cancer is refractory adenocarcinoma of esophagogastric junction.

In some embodiments of the present application, the esophagogastric junction cancer is recurrent and/or metastatic esophagogastric junction cancer. In some embodiments of the present application, the esophagogastric junction cancer is recurrent and/or metastatic adenocarcinoma of esophagogastric junction.

In some embodiments of the present application, the esophagogastric junction cancer is recurrent and/or metastatic advanced esophagogastric junction cancer. In some embodiments of the present application, the esophagogastric junction cancer is recurrent and/or metastatic advanced adenocarcinoma of esophagogastric junction.

In some embodiments of the present application, the esophagogastric junction cancer is unresectable locally advanced or metastatic adenocarcinoma of esophagogastric junction.

In some embodiments of the present application, the patient with esophagogastric junction cancer has not previously received drug therapy, or has previously received drug therapy but has failed or was intolerant to the therapy.

In some embodiments of the present application, the drug therapy includes chemotherapy, targeted drug therapy, and immunotherapy.

### Regimen for pharmaceutical combination

In some embodiments of the present application, in the use or treatment method described above, the anti-PD-L1 antibody and anlotinib or the pharmaceutically acceptable salt thereof are each in the form of a pharmaceutical composition and can be administered simultaneously, nonsimultaneously, or sequentially. In some embodiments of the present application, the anti-PD-L1 antibody, anlotinib or the pharmaceutically acceptable salt thereof, and the third therapeutic agent are each in the form of a pharmaceutical composition and can be administered simultaneously, nonsimultaneously, or sequentially. In some embodiments of the present application, the anti-PD-L1 antibody, anlotinib or the pharmaceutically acceptable salt thereof, oxaliplatin, and capecitabine are each in the form of a pharmaceutical composition and can be administered simultaneously, nonsimultaneously, or sequentially.

In some embodiments of the present application, in the use or treatment method described above, the anti-PD-L1 antibody and anlotinib are separately administered at intervals. In some embodiments, the antibody and anlotinib are separately administered on the same regimen or different regimens. In some embodiments, the antibody and anlotinib are separately administered on different regimens. In some embodiments of the present application, the anti-PD-L1 antibody, anlotinib or the pharmaceutically acceptable salt thereof, and the third therapeutic agent are separately administered at intervals and are separately administered on different regimens. In some embodiments of the present application, the anti-PD-L1 antibody, anlotinib or the pharmaceutically acceptable salt thereof, oxaliplatin, and capecitabine are separately administered at intervals and are separately administered on different regimens.

In some embodiments of the present application, in the use or treatment method described above, the anti-PD-L1 antibody can be administered once every 1 week (q1w), once every 2 weeks (q2w), once every 3 weeks (q3w), or once every 4 weeks (q4w). In a specific embodiment, the anti-PD-L1 antibody is administered once every 3 weeks. In some embodiments, the anti-PD-L1 antibody is administered at a dose of 600-2400 mg each time. Anlotinib or the pharmaceutically acceptable salt thereof can be administered at a dose of 6 mg, 8 mg, 10 mg, or 12 mg once daily on a regimen of consecutively 2-week treatment and then 1-week interruption.

Oxaliplatin can be administered once every 2 weeks (q2w) or once every 3 weeks (q3w). In a specific embodiment, oxaliplatin is administered once every 3 weeks. In some embodiments, oxaliplatin is administered at a dose of 85-130 mg/m² each time.

Capecitabine can be administered at a single dose of 1000-1250 mg/m² twice daily (once in the morning and once in the evening) on a regimen of consecutively 2-week treatment and then 1-week interruption.

In some embodiments, the anti-PD-L1 antibody and anlotinib or the pharmaceutically acceptable salt thereof each have the same or different treatment cycles. In some specific embodiments, the anti-PD-L1 antibody and anlotinib have the same treatment cycle, e.g., a 1-week, 2-week, 3-week or 4-week treatment cycle. In some embodiments, the anti-PD-L1 antibody, anlotinib or the pharmaceutically acceptable salt thereof, and the third therapeutic agent each have the same or different treatment cycles. In some embodiments, the anti-PD-L1 antibody, anlotinib or the pharmaceutically acceptable salt thereof, oxaliplatin, and capecitabine each have the same or different treatment cycles. In some specific embodiments, the anti-PD-L1 antibody, anlotinib or the pharmaceutically acceptable salt thereof, oxaliplatin, and capecitabine have the same treatment cycle, e.g., a 1-week, 2-week, 3-week or 4-week treatment cycle.

In some embodiments of the present application, the use or treatment method comprises: administering the anti-PD-L1 antibody, anlotinib or the pharmaceutically acceptable salt thereof, and the third therapeutic agent for an initial treatment; and then optionally administering the anti-PD-L1 antibody, and anlotinib or the pharmaceutically acceptable salt thereof for a maintenance treatment. In some embodiments, the anti-PD-L1 antibody, anlotinib or the pharmaceutically acceptable salt thereof, and the third therapeutic agent have the same treatment cycle, e.g., a 1-week, 2-week, 3-week or 4-week treatment cycle. In some embodiments, 3 weeks (21 days) are counted as one treatment cycle. In some embodiments, the initial treatment comprises 0 to 10 treatment cycles, preferably 1 to 10 treatment cycles, more preferably 1 to 6 treatment cycles, and most preferably 6 treatment cycles. In some embodiments, the initial treatment comprises 0 to 10 consecutive treatment cycles, preferably 1 to 10 consecutive treatment cycles, more preferably 1 to 6 consecutive treatment cycles, and most preferably 6 consecutive treatment cycles, from the first dose.

In some embodiments of the present application, in the use or treatment method, 3 weeks (21 days) are counted as one treatment cycle, the anti-PD-L1 antibody is administered on the first day of each cycle, and anlotinib or the pharmaceutically acceptable salt thereof is administered once daily on days 1-14 of each cycle. In some embodiments of the present application, in the use or treatment method, 21 days are counted as one treatment cycle, the anti-PD-L1 antibody and oxaliplatin are administered on the first day of each cycle, anlotinib or the pharmaceutically acceptable salt thereof is administered once daily on days 1-14 of each cycle, and capecitabine is administered twice daily on days 1-14 of each cycle.

In some embodiments of the present application, the use or treatment method comprises: with every 3 weeks (21 days) counted as one treatment cycle, administering the anti-PD-L1 antibody and oxaliplatin on the first day of each cycle and administering anlotinib or the pharmaceutically acceptable salt thereof and capecitabine on days 1-14 of each cycle for the initial treatment; and then optionally, with every 3 weeks counted as one treatment cycle, administering the anti-PD-L1 antibody on the first day of each cycle and administering anlotinib or the pharmaceutically acceptable salt thereof on days 1-14 of each cycle for the maintenance treatment. In some embodiments, the initial treatment comprises 0 to 10 treatment cycles, preferably 1 to 10 treatment cycles, more preferably 1 to 6 treatment cycles, and most preferably 6 treatment cycles. In some embodiments, the initial treatment comprises 0 to 10 consecutive treatment cycles, preferably 1 to 10 consecutive treatment cycles, more preferably 1 to 6 consecutive treatment cycles, and most preferably 6 consecutive treatment cycles, from the first dose.

In some embodiments of the present application, the use or treatment method comprises at least 1 to 10 treatment cycles of initial treatment (the anti-PD-L1 antibody, anlotinib or the pharmaceutically acceptable salt thereof, oxaliplatin, and capecitabine), preferably 1 to 6 treatment cycles of initial treatment. After the initial treatment is completed, a maintenance treatment (the anti-PD-L1 antibody, and anlotinib or the pharmaceutically acceptable salt thereof) is performed until the patient is intolerant or the disease progresses.

In some embodiments of the present application, in the use or treatment method, the anti-PD-L1 antibody can be administered to a subject at a dose selected from the group consisting of 0.01 to 40 mg/kg, 0.1 to 30 mg/kg, 0.1 to 20 mg/kg, 0.1 to 15 mg/kg, 0.1 to 10 mg/kg, 1 to 15 mg/kg, 1 to 20 mg/kg, 1 to 3 mg/kg, 3 to 10 mg/kg, 3 to 15 mg/kg, 3 to 20 mg/kg, 3 to 30 mg/kg, 10 to 20 mg/kg, and 15 to 20 mg/kg, or administered to a subject at a dose of 60 mg to 2400 mg, 90 mg to 1800 mg, 120 mg to 1500 mg, 300 mg to 900 mg, 600 mg to 900 mg, 300 mg to 1200 mg, 600 mg to 1200 mg, or 900 mg to 1200 mg.

In some embodiments for the use or treatment method, 21 days are counted as one treatment cycle, 1200 mg of the anti-PD-L1 antibody is administered on the first day of each cycle, and 6 mg, 8 mg, 10 mg and/or 12 mg of anlotinib or the pharmaceutically acceptable salt thereof is administered daily on days 1-14 of each cycle. In some embodiments for the use or treatment method, 21 days are counted as one treatment cycle, 1200 mg of the anti-PD-L1 antibody and oxaliplatin at a dose of 130 mg/m² are administered on the first day of each cycle, 6 mg, 8 mg, 10 mg and/or 12 mg of anlotinib or the pharmaceutically acceptable salt thereof is administered daily on days 1-14 of each cycle, and capecitabine can be administered at a single dose of 1000 mg/m² twice daily (once in the morning and once in the evening) on days 1-14 of each cycle.

In some embodiments, with every 3 weeks (21 days) counted as one treatment cycle, 1200 mg of the anti-PD-L1 antibody and oxaliplatin at a dose of 130 mg/m² are administered on the first day of each cycle, 6 mg, 8 mg, 10 mg and/or 12 mg of anlotinib or the pharmaceutically acceptable salt thereof is administered once daily on days 1-14 of each cycle, and capecitabine can be administered at a single dose of 1000 mg/m² twice daily (once in the morning and once in the evening) on days 1-14 of each cycle, so as to perform the initial treatment; and then optionally, with every 3 weeks counted as one treatment cycle, 1200 mg of the anti-PD-L1 antibody is administered on the first day of each cycle, and 6 mg, 8 mg, 10 mg and/or 12 mg of anlotinib or the pharmaceutically acceptable salt thereof is administered once daily on days 1-14 of each cycle, so as to perform the maintenance treatment, until the patient is intolerant or the disease progresses. In some embodiments, the initial treatment comprises 0 to 10 treatment cycles, preferably 1 to 10 treatment cycles, more preferably 1 to 6 treatment cycles, and most preferably 6 treatment cycles. In some embodiments, the initial treatment comprises 0 to 10 consecutive treatment cycles, preferably 1 to 10 consecutive treatment cycles, more preferably 1 to 6 consecutive treatment cycles, and most preferably 6 consecutive treatment cycles, from the first dose.

### Administration

The content below is not intended to limit the administration of the pharmaceutical combination disclosed herein. The components in the pharmaceutical combination disclosed herein can be administered independently, or some or all of the components are co-administered in various proper routes, including, but not limited to, oral administration or parenteral administration (by intravenous, intramuscular, local or subcutaneous routes). In some embodiments, the components in the pharmaceutical combination disclosed herein can be administered independently, or some or all of the components are co-administered by means of oral administration or injection, for example, intravenous injection or intraperitoneal injection.

The components in the pharmaceutical combination disclosed herein can be independent suitable dosage forms, or some or all of the components are co-formulated in a suitable dosage form, including, but not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, dispersant and dosage forms of sustained-release formulations for oral or non-oral administration.

The components in the pharmaceutical combination disclosed herein can be formulated independently, or some or all of the components are co-formulated with a pharmaceutically acceptable carrier and/or excipient.

In some embodiments, the pharmaceutical combination disclosed herein can safely and effectively treat gastric carcinoma and/or esophagogastric junction cancer.

In some embodiments, the pharmaceutical combination disclosed herein can safely and effectively treat non-HER2-positive gastric carcinoma and/or esophagogastric junction cancer. In some embodiments, the pharmaceutical combination disclosed herein can safely and effectively treat non-HER2-positive gastric carcinoma and/or adenocarcinoma of esophagogastric junction.

In some embodiments, in patients with gastric carcinoma and/or esophagogastric junction cancer, the pharmaceutical combination disclosed herein achieves a DCR (disease control rate) of up to or greater than 60%, up to or greater than 70%, up to or greater than 80%, up to or greater than 90%, up to or greater than 95%, or even up to 100%.

In some embodiments, after receiving the initial treatment with the pharmaceutical combination disclosed herein, patients with gastric carcinoma and/or esophagogastric junction cancer, particularly patients with non-HER2-positive gastric carcinoma and/or esophagogastric junction cancer, more particularly patients with non-HER2-positive gastric carcinoma and/or adenocarcinoma of esophagogastric junction, have a DCR of up to or greater than 60%, up to or greater than 70%, up to or greater than 80%, up to or greater than 90%, or up to or greater than 95%. Then, after receiving the maintenance treatment with the pharmaceutical combination disclosed herein, the patients have a DCR of up to or greater than 60%, up to or greater than 70%, up to or greater than 80%, up to or greater than 90%, up to or greater than 95%, or even up to 100%.

In some embodiments, in patients with gastric carcinoma and/or esophagogastric junction cancer, the pharmaceutical combination disclosed herein achieves an ORR (objective response rate) of up to or greater than 40%, up to or greater than 55%, up to or greater than 65%, up to or greater than 75%, up to or greater than 80%, up to or greater than 85%, or up to or greater than 90%.

In some embodiments, after receiving the initial treatment with the pharmaceutical combination disclosed herein, patients with gastric carcinoma and/or esophagogastric junction cancer, particularly patients with non-HER2-positive gastric carcinoma and/or esophagogastric junction cancer, more particularly patients with non-HER2-positive gastric carcinoma and/or adenocarcinoma of esophagogastric junction, have an ORR of up to or greater than 40%, up to or greater than 55%, up to or greater than 65%, up to or greater than 75%, or up to or greater than or 80%. Then, after receiving the maintenance treatment with the pharmaceutical combination disclosed herein, the patients have an ORR of up to or greater than 40%, up to or greater than 55%, up to or greater than 65%, up to or greater than 75%, up to or greater than 80%, up to or greater than 85%, or up to or greater than or 90%.

In some embodiments, the pharmaceutical combination disclosed herein can significantly prolong PFS (progression-free survival) and OS (overall survival) of patients with gastric carcinoma and/or esophagogastric junction cancer. In some embodiments, the PFS is more than 6 months, or is up to or more than 7 months, up to or more than 8 months, up to or more than 9 months, up to or more than 10 months, up to or more than 11 months, or up to or more than 12 months.

In some embodiments, the pharmaceutical combination disclosed herein has a synergistic effect and good safety when used to treat gastric carcinoma and/or esophagogastric junction cancer.

In some embodiments, the ORR is calculated according to the ratio of the number of objective response cases (PR + CR) evaluated by IRC to the total number of cases and 95% CI.

### Definitions and explanations

Unless otherwise stated, the following terms used in the present application shall have the following meanings. A certain term, unless otherwise specifically defined, should not be considered uncertain or unclear, but construed according to its common meaning in the field. When referring to a trade name, it is intended to refer to its corresponding commercial product or its active ingredient.

Herein, unless otherwise stated, the amount of anlotinib or a pharmaceutically acceptable salt thereof mentioned herein refers to the amount of the active ingredient anlotinib free base.

Unless otherwise stated, the term "dose" refers to a dose administered to a patient without considering the weight or body surface area (BSA) of the patient. For example, a 60 kg human and a 100 kg human will receive the same dose of antibody (e.g., 240 mg of anti-PD-1 antibody). In addition, it will be understood, based on the general knowledge of those skilled in the art, that mg/m² refers to the dose of a drug used per square meter of body surface area of a subject.

As used herein, the term "pharmaceutical combination" refers to a combination of two or more active ingredients (administered as the respective active ingredients themselves, or as their respective derivatives like pharmaceutically acceptable salts or esters, prodrugs, or compositions) that are administered simultaneously or sequentially. The active substances can be administered to a subject simultaneously or sequentially in any order as a single formulation.

As used herein, the term "antibody" refers to a binding protein having at least one antigen-binding domain. The antibody and the fragment thereof of the present application can be an intact antibody or any fragment thereof. Thus, the antibody and the fragment thereof of the present application include a monoclonal antibody or a fragment thereof and an antibody variant or a fragment thereof, as well as an immunoconjugate. Examples of the antibody fragment include a Fab fragment, a Fab' fragment, an F(ab')₂ fragment, an Fv fragment, an isolated CDR region, a single chain Fv molecule (scFv), an Fd fragment, and other antibody fragments known in the art. The antibody and the fragment thereof may also include a recombinant polypeptide, a fusion protein, and a bispecific antibody. The anti-PD-L1 antibody and the fragment thereof described herein can be of IgG1, IgG2, IgG3, or IgG4 isotype. The term "isotype" refers to the class of antibodies encoded by the heavy chain constant region gene. In one embodiment, the anti-PD-L1 antibody and the fragment thereof described herein are of the IgG1 or IgG4 isotype. The anti-PD-L1 antibody and the fragment thereof of the present application can be derived from any species including, but not limited to, mouse, rat, rabbit, primate, llama, and human. The anti-PD-L1 antibody and the fragment thereof can be a chimeric antibody, a humanized antibody, or an intact human antibody. In one embodiment, the anti-PD-L1 antibody is an antibody produced by a hybridoma cell line derived from a mouse. Thus, in one embodiment, the anti-PD-L1 antibody is a murine antibody. In another embodiment, the anti-PD-L1 antibody is a chimeric antibody. In another embodiment, the chimeric antibody is a mouse-human chimeric antibody. In another embodiment, the antibody is a humanized antibody. In another embodiment, the antibody is derived from a murine antibody and is humanized.

The term "humanized antibody" refers to an antibody comprising complementarity determining regions (CDRs) derived from a non-human antibody, and framework and constant regions derived from a human antibody. For example, an anti-PD-L1 antibody described herein may comprise CDRs derived from one or more murine antibodies as well as human framework and constant regions. Thus, in one embodiment, the humanized antibody described herein binds to the same epitope on PD-L1 as the murine antibody from which the CDRs of the humanized antibody are derived. In some embodiments, the anti-PD-L1 antibody is a humanized antibody. Additional anti-PD-L1 antibodies or variants thereof comprising the heavy and light chain CDRs disclosed herein can be generated using any human framework sequences, and are also included in the present application. In one embodiment, framework sequences suitable for use in the present application include those similar in structure to the framework sequences disclosed herein. Additional modifications may be made in the framework regions to improve the properties of the antibodies provided herein. Such additional framework modifications may include: chemical modifications, point mutations for reducing immunogenicity or removing T cell epitopes, or modifications reverting the mutations to residues in original germline sequences. In some embodiments, such modifications include those corresponding to the mutations exemplified herein, including reversions to germline sequences. For example, in one embodiment, one or more amino acids in the human VH and/or VL framework regions of the humanized antibodies described herein are reverted to the corresponding amino acids in the parent murine antibodies. For example, for the VH and VL of humanized 5G11 and humanized 13C5 antibodies, several sites of framework amino acids of the template human antibodies described above are reverted to the corresponding amino acid sequences in the mouse 5G11 and 13C5 antibodies. In one embodiment, the amino acids at positions 53, 60 and/or 67 of the light chain variable region are reverted to the corresponding amino acids found at the positions in mouse 5G11 or 13C5 light chain variable region. In another embodiment, the amino acids at positions 24, 28, 30, 49, 73, 83 and/or 94 of the heavy chain variable region are reverted to the corresponding amino acids found at the positions in mouse 5G11 or 13C5 heavy chain variable region. In one embodiment, the humanized 5G11 antibody comprises: a light chain variable region, wherein the amino acid at position 60 is mutated from Ser (S) to Asp (D) and the amino acid at position 67 is mutated from Ser (S) to Tyr (Y); and a heavy chain variable region, wherein the amino acid at position 24 is mutated from Phe (F) to Val (V), the amino acid at position 49 is mutated from Ala (A) to Gly (G), the amino acid at position 73 is mutated from Thr (T) to Asn (N), and the amino acid at position 83 is mutated from Thr (T) to Asn (N). In one embodiment, the humanized 13C5 antibody comprises: a light chain variable region, wherein the amino acid at position 53 is mutated from Tyr (Y) to Lys (K); and a heavy chain variable region, wherein the amino acid at position 28 is mutated from Thr (T) to Ile (I), the amino acid at position 30 is mutated from Ser (S) to Arg (R), the amino acid at position 49 is mutated from Ser (S) to Ala (A), and the amino acid at position 94 is mutated from Tyr (Y) to Asp (D). Additional or alternative reverse mutations can be made in the framework regions of the humanized antibodies provided herein to improve the properties of the antibodies. The present application also includes humanized antibodies that bind to PD-L1 and comprise framework modifications corresponding to the exemplary modifications disclosed herein relative to any suitable framework sequence, as well as other framework modifications that otherwise improve antibody properties.

For the isolated antibody or the fragment thereof that binds to PD-L1 described herein, the antibody can be produced by a hybridoma selected from the group consisting of the hybridomas designated herein as 13C5 and 5G11. Accordingly, the antibody described herein further includes hybridomas 13C5 and 5G11, and any hybridomas that produce the antibodies disclosed herein. The present application further provides isolated polynucleotides encoding the antibodies and the fragments thereof disclosed herein. The present application also includes expression vectors comprising the isolated polynucleotides, and host cells comprising the expression vectors.

The "isolated antibody" refers to an antibody that is substantially free of other antibodies having different antigenic specificities (e.g., an isolated antibody that specifically binds to PD-1 is substantially free of antibodies that specifically bind to antigens apart from PD-1). However, an isolated antibody that specifically binds to PD-1 may have cross-reactivity with other antigens (such as PD-1 molecules from different species). Furthermore, the isolated antibody may be substantially free of other cellular materials and/or chemicals.

The term "monoclonal antibody" ("mAb") refers to a non-naturally occurring preparation of antibody molecules of an individual molecule component (i.e., antibody molecules whose base sequences are substantially identical and which exhibit a single binding specificity and affinity for a particular epitope). mAb is an example of the isolated antibody. mAbs can be produced by hybridoma techniques, recombinant techniques, transgenic techniques, or other techniques known to those skilled in the art.

The antibody or the antigen-binding fragment thereof described herein is specific for PD-L1. In one embodiment, the antibody or/and the fragment thereof is specific for PD-L1. In one embodiment, the antibody or the fragment thereof described herein binds to human or primate PD-L1, but does not bind to PD-L1 from any other mammals. In another embodiment, the antibody or/and the fragment thereof does not bind to mouse PD-L1. The terms "human PD-L1", "hPD-L1", "huPD-L1", and the like are used interchangeably herein and refer to human PD-L1 and variants or isotypes of human PD-L1. The terms "specific", "specificity" and "specifically" refer to that the antibody and the fragment thereof bind to PD-L1 with greater affinity than any other targets.

The term "treat", "treating" or "treatment" usually refers to acquiring needed pharmacological effect and/or physiological effect. In terms of partially or fully stabilizing or curing the disease and/or a side effect of the disease, the effect can be therapeutic. As used herein, "treat", "treating" or "treatment" encompasses any treatment of a disease in a patient, including (a) inhibiting a symptom of the disease, i.e., blocking the progression of the disease; or (b) alleviating a symptom of the disease, i.e., causing remission of the disease or the symptom.

The term "effective amount" refers to an amount of the compound disclosed herein for (i) treating a specific disease, condition or disorder; (ii) alleviating, ameliorating or eliminating one or more symptoms of a specific disease, condition or disorder, or (iii) preventing or delaying onset of one or more symptoms of the specific disease, condition or disorder described herein. The amount of active substance (e.g., the antibody or compound of the present application) constituting the "therapeutically effective amount" may vary according to factors such as the disease state, age, sex, and weight of the individual, and the ability of a therapeutic agent or a combination of therapeutic agents to elicit a desired response in the individual. The effective amount may also be determined routinely by those skilled in the art in accordance with their knowledge and the present disclosure.

The terms "administer", "administration" and "administering" are used interchangeably and refer to physically introducing the composition comprising a therapeutic agent to an entity using any of a variety of methods and delivery systems known to those skilled in the art. Routes of administration of immune checkpoint inhibitors (e.g., an anti-PD-1 antibody or an anti-PD-L1 antibody) include parenteral routes of administration (including but not limited to intravenous, intramuscular, subcutaneous, intraperitoneal, spinal, or other parenteral routes of administration), for example, by injection or infusion. In some embodiments, the phrase "parenteral administration" or "administered parenterally" as used herein is used interchangeably, and typically refers to modes of administration apart from enteral and local administration, typically by injection, including but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and *in vivo* electroporation. In some embodiments, the immune checkpoint inhibitor (e.g., an anti-PD-1 antibody or an anti-PD-L1 antibody) is administered by a non-parenteral route, and in some embodiments, by oral administration; other non-parenteral routes include local, epidermal or mucosal administration route, e.g., intranasal, intravaginal, intrarectal, sublingual or local administration route. Administration may also be performed, e.g., once, multiple times, and/or over one or more extended periods of time.

The term "pharmaceutically acceptable" is used herein for those compounds, materials, compositions, and/or dosage forms that are suitable for use in contact with the tissues of human beings and animals without excessive toxicity, irritation, allergic response, or other problems or complications.

The term "pharmaceutically acceptable salt" includes salts formed from a free base and an acid and salts formed from an acid and a free base, for example, hydrochloride, hydrobromide, nitrate, sulfate, phosphate, formate, acetate, trifluoroacetate, fumarate, oxalate, maleate, citrate, succinate, mesylate, benzenesulfonate or *p*-methylbenzenesulfonate; in some embodiments, the pharmaceutically acceptable salt is hydrochloride, hydrobromide, sulfate, formate, acetate, trifluoroacetate, fumarate, maleate, mesylate, *p*-methylbenzenesulfonate, sodium salt, potassium salt, ammonium salt and amino acid salt, or the like. In the present application, in forming a pharmaceutically acceptable salt, the acid and the free base are in a molar ratio of 1:0.2-1:5; in some embodiments, the molar ratio is 1:0.5, 1:1, 1:2, 1:3, 1:4, 1:5, 1:6, 1:7, or 1:8.

As used herein, the terms "subject" and "patient" are used interchangeably. In some embodiments, the term "subject" or "patient" refers to a mammal. In some embodiments, the subject or patient is a mouse. In some embodiments, the subject or patient is a human.

The term "unit dose" refers to the smallest unit of packaging containing a certain quantity of pharmaceutical product; for example, in a box of seven capsules, each capsule is a unit dose; or a vial of injection is a unit dose. The term "multiple dose" consists of multiple unit doses.

The term "pharmaceutical composition" refers to a mixture consisting of one or more of the active ingredients or pharmaceutical combinations thereof of the present application and a pharmaceutically acceptable excipient. The pharmaceutical composition is intended to facilitate the administration of the compound or the pharmaceutical combination thereof of the present application to a subject.

The term "fluorouracil anti-tumor drug" refers to a drug containing a fluorinated derivative of uracil having anti-tumor activity, or a drug convertible *in vivo* into 5-fluorouracil. The fluorouracil anti-tumor drug includes, but is not limited to, one or more selected from the group consisting of capecitabine, 5-fluorouracil, difuradin, doxifluridine, trifluridine, tegafur, carmofur, tegafur-gimeracil-oteracil potassium, and uracil-tegafur.

The term "platinum anti-tumor drug" refers to a drug containing a platinum complex having anti-tumor activity. The platinum anti-tumor drug includes, but is not limited to, one or more selected from the group consisting of miriplatin, cisplatin, carboplatin, dicycloplatin, nedaplatin, oxaliplatin, lobaplatin, triplatin tetranitrate, phenanthriplatin, picoplatin, and satraplatin.

The term "paclitaxel anti-tumor drug" refers to a drug containing paclitaxel and a derivative thereof having anti-tumor activity. The paclitaxel anti-tumor drug includes, but is not limited to, one or more selected from the group consisting of paclitaxel, paclitaxel liposome, albumin-bound paclitaxel, and docetaxel.

The term "esophagogastric junction cancer" may also be referred to as gastroesophageal junction cancer.

The term "adenocarcinoma of esophagogastric junction (AEG)" may also be referred to as gastroesophageal junction adenocarcinoma.

The term "initial treatment" refers to administration of an anti-PD-L1 antibody, and anlotinib or a pharmaceutically acceptable salt thereof, and a third therapeutic agent to a patient in a repeated cycle.In some embodiments, the initial treatment is administration of an anti-PD-L1 antibody, anlotinib or a pharmaceutically acceptable salt thereof, capecitabine, and oxaliplatin to a patient in a repeated cycle.

The term "maintenance treatment" refers to administration of an anti-PD-L1 antibody, and anlotinib or a pharmaceutically acceptable salt thereof to a patient in a repeated cycle.

The term "repeated cycle" refers to 0, 1, or more consecutive treatment cycles.

As used herein, unless otherwise stated, the terms "comprise", "comprises" and "comprising" or equivalents thereof are open-ended statements and mean that elements, components and steps that are not specified may be included in addition to those listed.

As used herein, unless otherwise stated, all numbers expressing the amounts of ingredients, measurements, or reaction conditions used herein are to be understood as being modified in all instances by the term "about". The term "about" when connected to a percentage may mean, for example, ± 0.1%, preferably, ± 0.05%, and more preferably, ± 0.01%.

Unless otherwise specified clearly herein, singular terms encompass plural referents, and vice versa. Similarly, unless otherwise specified clearly herein, the word "or" is intended to include "and".

All patents, patent applications and other publications are explicitly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the common knowledge in the art.

### DETAILED DESCRIPTION

For clarity, the present application is further described with the following examples, which are, however, not intended to limit the scope of the present application. All reagents used in the present application are commercially available and can be used without further purification. In the examples, the anti-PD-L1 antibody was prepared as described in WO2016022630, and after affinity chromatography, an eluate containing the antibody was obtained by conventional antibody purification methods.

### Example 1. Phase II Clinical Trial for Gastric Carcinoma and/or Esophagogastric Junction Cancer

Subjects with gastric carcinoma and/or esophagogastric junction cancer who met the inclusion criteria were first subjected to 6 treatment cycles of initial treatment (a four-drug combination regimen with an anti-PD-L1 antibody, anlotinib hydrochloride, oxaliplatin, and capecitabine), and then subjected to a maintenance treatment (a two-drug combination regimen with an anti-PD-L1 antibody and anlotinib hydrochloride) until disease progression, occurrence of intolerable toxicity, withdrawal of informed consent, lost to follow-up or death, or other conditions in which the investigator judged that the treatment should be discontinued, whichever occurred first.

### 1.1 Primary inclusion criteria

1) With unresectable locally advanced or metastatic gastric carcinoma or gastroesophageal junction adenocarcinoma (including signet-ring cell carcinoma, mucinous adenocarcinoma, and hepatoid adenocarcinoma) confirmed to be HER2/neu-negative (or have indeterminate HER2/neu status) by pathological (histological or cytological) examination;
2) Time from the end of previous (new) adjuvant chemotherapy/adjuvant radiotherapy to disease recurrence > 6 months;
3) According to RECIST version 1.1, having at least one measurable lesion or evaluable lesion, wherein the measurable lesion should not have received local treatments such as radiotherapy (lesions located within the area that has undergone previous radiotherapy may also be selected as target lesions if they are confirmed to have progressed and meet RECIST 1.1 criteria);
4) Aged: 18-75 years; and
5) ECOG PS score: 0-1; expected survival time ≥ 3 months.

### 1.2 Test drug

Anti-PD-L1 antibody injection hu5G11-hIgG1: 1200 mg of an anti-PD-L1 antibody injection was diluted to 100 mL with normal saline, and the diluted drug was administered in about 40 min by infusion (the infusion system was flushed with normal saline before and after infusion). The anti-PD-L1 antibody injection was administered on the first day, and once every 21 days, that is, 21 days were counted as one treatment cycle (d1/q3w).

Anlotinib hydrochloride capsule (anlotinib dihydrochloride as active ingredient): once daily (orally taken before breakfast on an empty stomach), 1 capsule (10 mg) each time. The oral administration was performed for 2 consecutive weeks and interrupted for 1 week, that is, 21 days were counted as one treatment cycle, and the drug was administered on days 1-14 of each cycle. Except in special circumstances, it was recommended to administer the drug at a fixed time every day (that is, the anlotinib hydrochloride capsule was administered according to the following regimen: 10 mg/qd, d1-14/q3w).

The investigator could adjust the dose of the anlotinib hydrochloride capsule, e.g., to 12 mg, 10 mg or 8 mg, according to the disease condition, safety and other aspects.

Oxaliplatin: administered at a single dose of 130 mg/m² by intravenous drip on the first day, and once every 21 days, that is, 21 days were counted as one treatment cycle (d1/q3w).

Capecitabine: administered at a single dose of 1000 mg/m² twice daily (orally, once in the morning and once in the evening) for 2 consecutive weeks and interrupted for 1 week, that is, 21 days were counted as one treatment cycle, and the drug was administered on days 1-14 of each cycle (d1-14/q3w).

### 1.3 Evaluation criteria

Evaluation criteria for safety: NCI CTC AE 5.0 criteria for the adverse events of the test drug.

Evaluation criteria for effectiveness: RECIST 1.1 and iRECIST criteria for the curative effect of the test drug. RECIST 1.1 criteria were used as the primary criteria, and iRECIST criteria were used to confirm the curative effect. That is, subjects determined to have progressive disease (PD) according to RECIST 1.1 criteria were further confirmed according to iRECIST criteria to determine whether to take further medication observation.

### 1.4 Endpoints

### Primary endpoint: objective response rate (ORR)

Secondary endpoint: (1) drug safety; (2) disease control rate (DCR); (3) objective response rate (ORR); (4) duration of response (DOR); and (5) progression-free survival (PFS) and overall survival (OS).

### 1.5 Results

Preliminary studies show that the subjects had an ORR of greater than 40%, even up to or greater than 90% after receiving the initial treatment (a four-drug combination regimen with an anti-PD-L1 antibody, anlotinib hydrochloride, oxaliplatin, and capecitabine), and achieved a further improvement in ORR after receiving the maintenance treatment (a two-drug combination regimen with an anti-PD-L1 antibody and anlotinib hydrochloride). There are data showing that, according to the statistics for best curative effect, among the 10 subjects who could be evaluated for curative effect, 4 subjects (4/10) were in complete response, 5 subjects (5/10) were in partial response, and 1 subject (1/10) had stable disease, achieving 90% ORR and 100% DCR. In addition, 3 subjects were withheld from treatment, and the preliminary median PFS exceeded 6 months. The treatment regimens in the test demonstrated surprising curative effects in the treatment of gastric carcinoma and/or adenocarcinoma of esophagogastric junction, and had safety and controllable adverse events. The test shows that the four-drug combination regimen with the anti-PD-L1 antibody, anlotinib hydrochloride, oxaliplatin and capecitabine and the two-drug combination regimen with the anti-PD-L1 antibody and anlotinib hydrochloride can safely and effectively treat gastric carcinoma and/or adenocarcinoma of esophagogastric junction to provide clinical benefits to the subjects, and the four-drug combination regimen can be used as a first-line treatment regimen for gastric carcinoma and/or adenocarcinoma of esophagogastric junction.

Those skilled in the art will recognize that the scope of the present application is not limited to the various embodiments and examples described above. Instead, various modifications, substitutions, or recombinations can be made without departing from the spirit of the present application, all of which fall within the protection scope of the present application.

## Claims

1. Use of a pharmaceutical combination comprising an anti-PD-L1 antibody, and anlotinib or a pharmaceutically acceptable salt thereof for preparing a medicament for treating gastric carcinoma and/or esophagogastric junction cancer.

2. The use according to claim 1, wherein the pharmaceutical combination further comprises at least one third therapeutic agent, wherein the third therapeutic agent is one or more selected from the group consisting of a fluorouracil anti-tumor drug, a platinum anti-tumor drug, and a paclitaxel anti-tumor drug.

3. The use according to claim 2, wherein the third therapeutic agent comprises a fluorouracil anti-tumor drug and/or a platinum anti-tumor drug, preferably capecitabine and/or oxaliplatin.

4. The use according to any one of claims 1-3, wherein the pharmaceutical combination comprises a first pharmaceutical combination administered to a patient in need thereof during an initial treatment and, optionally, a second pharmaceutical combination administered to a patient in need thereof during a maintenance treatment, wherein the first pharmaceutical combination comprises an anti-PD-L1 antibody, and anlotinib or a pharmaceutically acceptable salt thereof, and at least one third therapeutic agent, and the second pharmaceutical combination comprises an anti-PD-L1 antibody, and anlotinib or a pharmaceutically acceptable salt thereof.

5. The use according to claim 4, wherein the initial treatment comprises 0 to 10 treatment cycles, preferably 1 to 10 treatment cycles, more preferably 1 to 6 treatment cycles, and most preferably 6 treatment cycles.

6. The use according to any one of claims 1-5, wherein the anti-PD-L1 antibody is prepared as a pharmaceutical composition, and the anti-PD-L1 antibody in the pharmaceutical composition is at a concentration of 10-60 mg/mL, or 10 mg/mL, 20 mg/mL, 30 mg/mL, 40 mg/mL, 50 mg/mL, or 60 mg/mL.

7. The use according to any one of claims 1-6, wherein the pharmaceutical combination is a formulation suitable for administration within a single treatment cycle (e.g., a treatment cycle of 21 days), and comprises: a pharmaceutical composition comprising 600-2400 mg of the anti-PD-L1 antibody and a pharmaceutical composition comprising 84-168 mg of anlotinib or the pharmaceutically acceptable salt thereof.

8. The use according to claim 7, wherein the formulation suitable for administration within a single treatment cycle further comprises: a pharmaceutical composition comprising 65-780 mg of oxaliplatin, and/or a pharmaceutical composition comprising 14000-168000 mg of capecitabine.

9. The use according to any one of claims 1-8, wherein the anti-PD-L1 antibody or the pharmaceutical composition thereof is administered at a single dose of 600 mg, 800 mg, 1000 mg, 1200 mg, 1400 mg, 1600 mg, 1800 mg, 2000 mg, 2200 mg, or 2400 mg, and anlotinib or the pharmaceutically acceptable salt thereof is administered at a single dose of 6-12 mg, or 6 mg, 8 mg, 10 mg, or 12 mg.

10. The use according to any one of claims 3-9, wherein oxaliplatin is administered at a dose of 130 mg/m².

11. The use according to any one of claims 3-10, wherein capecitabine is administered at a single dose of 1000 mg/m² twice daily.

12. The use according to any one of claims 1-11, wherein the anti-PD-L1 antibody, and anlotinib or the pharmaceutically acceptable salt thereof can be administered simultaneously, nonsimultaneously, or sequentially.

13. The use according to any one of claims 2-12, wherein the anti-PD-L1 antibody, anlotinib or the pharmaceutically acceptable salt thereof, and the third therapeutic agent can be administered simultaneously, nonsimultaneously, or sequentially.

14. The use according to any one of claims 1-13, wherein every 3 weeks is counted as one treatment cycle, the anti-PD-L1 antibody is administered on the first day of each cycle, and anlotinib or the pharmaceutically acceptable salt thereof is administered on days 1-14 of each cycle.

15. The use according to any one of claims 3-14, wherein every 3 weeks is counted as one treatment cycle, oxaliplatin is administered on the first day of each cycle, and capecitabine is administered on days 1-14 of each cycle.

16. The use according to any one of claims 1-15, wherein the gastric carcinoma comprises tubular adenocarcinoma, parietal cell adenocarcinoma, mixed adenocarcinoma, papillary adenocarcinoma, mucoepidermoid carcinoma, mucinous adenocarcinoma, signet-ring cell carcinoma, poorly cohesive carcinoma, hepatoid adenocarcinoma, and paneth cell carcinoma.

17. The use according to any one of claims 1-16, wherein the gastric carcinoma and/or esophagogastric junction cancer is non-HER2-positive gastric carcinoma and/or adenocarcinoma of esophagogastric junction;
or the gastric carcinoma and/or esophagogastric junction cancer is advanced and/or refractory and/or recurrent and/or metastatic gastric carcinoma and/or adenocarcinoma of esophagogastric junction.

18. A method for treating gastric carcinoma and/or esophagogastric junction cancer, comprising: administering to a patient in need thereof a therapeutically effective amount of an anti-PD-L1 antibody, and anlotinib or a pharmaceutically acceptable salt thereof.

19. The method according to claim 18, further comprising administering to a patient in need thereof a therapeutically effective amount of at least one third therapeutic agent, wherein the third therapeutic agent is one or more of a fluorouracil anti-tumor drug, a platinum anti-tumor drug, and a paclitaxel anti-tumor drug.

20. The method according to claim 19, wherein the third therapeutic agent comprises a fluorouracil anti-tumor drug and/or a platinum anti-tumor drug, preferably capecitabine and/or oxaliplatin.

21. The method according to claim 19 or 20, comprising: administering the anti-PD-L1 antibody, and anlotinib or the pharmaceutically acceptable salt thereof, and the third therapeutic agent for an initial treatment; and then optionally administering the anti-PD-L1 antibody, and anlotinib or the pharmaceutically acceptable salt thereof for a maintenance treatment.

22. The method according to claim 21, wherein the initial treatment comprises 0 to 10 treatment cycles, preferably 1 to 10 treatment cycles, more preferably 1 to 6 treatment cycles, and most preferably 6 treatment cycles.

23. The method according to any one of claims 18-22, wherein the anti-PD-L1 antibody is administered at a single dose of 600-2400 mg, or 600 mg, 800 mg, 1000 mg, 1200 mg, 1400 mg, 1600 mg, 1800 mg, 2000 mg, 2200 mg, or 2400 mg.

24. The method according to any one of claims 18-23, wherein anlotinib or the pharmaceutically acceptable salt thereof is administered at a single dose of 6-12 mg, or 6 mg, 8 mg, 10 mg, or 12 mg.

25. The method according to any one of claims 20-24, wherein oxaliplatin is administered at a dose of 130 mg/m².

26. The method according to any one of claims 20-25, wherein capecitabine is administered at a single dose of 1000 mg/m² twice daily.

27. The method according to any one of claims 18-26, wherein the anti-PD-L1 antibody and anlotinib or the pharmaceutically acceptable salt thereof are each in the form of a pharmaceutical composition and can be administered simultaneously, nonsimultaneously, or sequentially.

28. The method according to any one of claims 19-27, wherein the anti-PD-L1 antibody, anlotinib or the pharmaceutically acceptable salt thereof, and the third therapeutic agent are each in the form of a pharmaceutical composition and can be administered simultaneously, nonsimultaneously, or sequentially.

29. The method according to any one of claims 18-28, wherein every 1 week, every 2 weeks, every 3 weeks, or every 4 weeks is counted as one treatment cycle.

30. The method according to any one of claims 18-29, wherein every 3 weeks is counted as one treatment cycle, the anti-PD-L1 antibody is administered on the first day of each cycle, and anlotinib or the pharmaceutically acceptable salt thereof is administered on days 1-14 of each cycle.

31. The method according to any one of claims 20-30, wherein every 3 weeks is counted as one treatment cycle, oxaliplatin is administered on the first day of each cycle, and capecitabine is administered on days 1-14 of each cycle.

32. The method according to any one of claims 18-31, wherein the gastric carcinoma and/or esophagogastric junction cancer is non-HER2-positive gastric carcinoma and/or adenocarcinoma of esophagogastric junction.

33. The method according to any one of claims 18-32, wherein the gastric carcinoma and/or esophagogastric junction cancer is advanced and/or refractory and/or recurrent and/or metastatic gastric carcinoma and/or adenocarcinoma of esophagogastric junction.

34. A pharmaceutical combination for treating gastric carcinoma and/or esophagogastric junction cancer, comprising: an anti-PD-L1 antibody, and anlotinib or a pharmaceutically acceptable salt thereof.

35. The pharmaceutical combination according to claim 34, further comprising at least one third therapeutic agent, wherein the third therapeutic agent is one or more of a fluorouracil anti-tumor drug, a platinum anti-tumor drug, and a paclitaxel anti-tumor drug, and preferably, the third therapeutic agent comprises capecitabine and/or oxaliplatin.

36. The pharmaceutical combination according to claim 34 or 35, packaged in a kit further comprising instructions for the treatment of gastric carcinoma and/or esophagogastric junction cancer.

37. The pharmaceutical combination according to any one of claims 34-36, comprising: a pharmaceutical composition comprising 600-2400 mg of the anti-PD-L1 antibody provided in a multiple-dose form, and a pharmaceutical composition comprising 6 mg, 8 mg, 10 mg, and/or 12 mg of anlotinib or the pharmaceutically acceptable salt thereof in a unit dose.

38. The pharmaceutical combination according to any one of claims 34-37, wherein the pharmaceutical composition of the anti-PD-L1 antibody is a solution for injection; and the pharmaceutical composition of anlotinib or the pharmaceutically acceptable salt thereof is an oral solid formulation.

39. The use according to any one of claims 1-17, or the method according to any one of claims 18-33, or the pharmaceutical combination according to any one of claims 34-38, wherein the anti-PD-L1 antibody comprises the following amino acid sequences: a heavy chain CDR1 region having at least 80% homology to an amino acid sequence set forth in SEQ ID NO: 1 or SEQ ID NO: 4; a heavy chain CDR2 region having at least 80% homology to an amino acid sequence set forth in SEQ ID NO: 2 or SEQ ID NO: 5; a heavy chain CDR3 region having at least 80% homology to an amino acid sequence set forth in SEQ ID NO: 3 or SEQ ID NO: 6; a light chain CDR1 region having at least 80% homology to an amino acid sequence set forth in SEQ ID NO: 7 or SEQ ID NO: 10; a light chain CDR2 region having at least 80% homology to an amino acid sequence set forth in SEQ ID NO: 8 or SEQ ID NO: 11; and a light chain CDR3 region having at least 80% homology to an amino acid sequence set forth in SEQ ID NO: 9 or SEQ ID NO: 12.

40. The use according to any one of claims 1-17, or the method according to any one of claims 18-33, or the pharmaceutical combination according to any one of claims 34-38, wherein the anti-PD-L1 antibody comprises the following amino acid sequences: a heavy chain CDR1 region selected from the group consisting of SEQ ID NO: 1 and SEQ ID NO: 4; a heavy chain CDR2 region selected from the group consisting of SEQ ID NO: 2 and SEQ ID NO: 5; a heavy chain CDR3 region selected from the group consisting of SEQ ID NO: 3 and SEQ ID NO: 6; a light chain CDR1 region selected from the group consisting of SEQ ID NO: 7 and SEQ ID NO: 10; a light chain CDR2 region selected from the group consisting of SEQ ID NO: 8 and SEQ ID NO: 11; and a light chain CDR3 region selected from the group consisting of SEQ ID NO: 9 and SEQ ID NO: 12.

41. The use according to any one of claims 1-17, or the method according to any one of claims 18-33, or the pharmaceutical combination according to any one of claims 34-38, wherein the anti-PD-L1 antibody comprises: a heavy chain CDR1 region having an amino acid sequence set forth in SEQ ID NO: 1; a heavy chain CDR2 region having an amino acid sequence set forth in SEQ ID NO: 2; a heavy chain CDR3 region having an amino acid sequence set forth in SEQ ID NO: 3; a light chain CDR1 region having an amino acid sequence set forth in SEQ ID NO: 7; a light chain CDR2 region having an amino acid sequence set forth in SEQ ID NO: 8; and a light chain CDR3 region having an amino acid sequence set forth in SEQ ID NO: 9.

42. The use according to any one of claims 1-17, or the method according to any one of claims 18-33, or the pharmaceutical combination according to any one of claims 34-38, wherein the anti-PD-L1 antibody comprises the following amino acid sequences: a heavy chain variable region having at least 80% homology to an amino acid sequence set forth in SEQ ID NO: 13 or SEQ ID NO: 14; and a light chain variable region having at least 80% homology to an amino acid sequence set forth in SEQ ID NO: 15 or SEQ ID NO: 16.

43. The use according to any one of claims 1-17, or the method according to any one of claims 18-33, or the pharmaceutical combination according to any one of claims 34-38, wherein the anti-PD-L1 antibody comprises: a heavy chain variable region of humanized antibodies selected from the group consisting of hu13C5-hIgG1, hu13C5-hIgG4, hu5G11-hIgG1 and hu5G11-hIgG4; and a light chain variable region of humanized antibodies selected from the group consisting of hu13C5-hIgG1, hu13C5-hIgG4, hu5G11-hIgG1 and hu5G11-hIgG4.
